# EUROPEAN PATENT APPLICATION

(11) **EP 1 217 080 A2**
(43) Date of publication of application: **26.06.2002**
(21) Application number: 01310567.1
(22) Date of filing: 18.12.2001
(51) Int. Cl.: C12Q 1/68

(54) **Methods, compositions and kits relating to cardiovascular disease**

(30) Priority: 22.12.2000 US 258072 P
(71) Applicant: Pfizer Products Inc., Groton, Connecticut 06340 (US)
(72) Inventor: Durham, Laura Kathryn, Pfizer Global Res. & Devel., Groton, Connecticut 06340 (US); Lira, Maruja Elma, Groton, Connecticut 06340 (US); Milos, Patrice Marie, Groton, Connecticut 06340 (US)
(74) Representative: Hayles, James Richard

(57) **Abstract**

The present invention relates to technologies for assessing a subjects susceptibility to development of a cardiovascular disorder. The invention further relates to methods for selecting therapeutics which will be optimally effective for treating patients suffering from a cardiovascular disorder. The invention includes methods, compositions and kits useful for determining a patients cholesteryl ester transfer protein (CETP) genotype.

SEQUENCE LISTING

<110> Pfizer Products Inc.

<120> METHODS, COMPOSITIONS AND KITS RELATING TO
      CARDIOVASCULAR DISEASE

<130> PC11028AJAK

<140> 60/258,072
<141> 2000-12-00

<160> 14

<170> PatentIn Ver. 3.1

<210> 1
<211> 1656
<212> DNA
<213> Homo sapiens

<400> 1
tgtctttttc tcatagtcat tgtattttgg cctctttcta tttatggcaa cagagagaga 60
aagcttattc ctagatatat gtatttaagt aaaaataaat gaattcatgg aaacatatta 120
agcaattatc cagataacat aagggatggc aaaaatggtg cagatggtgg aggggagaca 180
agtagaagtt ggggtgctct tgttgaatgt ctggctctga actctagagg aggccgcagg 240
ggctgggcag gaaggaggtg aatctctggg gccaggaaga ccctgctgcc cggaagagcc 300
tcatgttccg tgggggctgg gcggacatac atatacgggc tccaggctga acggctcggg 360
ccacttacac accactgcct gataaccatg ctggctgcca cagtcctgac cctggccctg 420
ctgggcaatg cccatgcctg ctccaaaggc acctcgcacg aggcaggcat cgtgtgccgc 480
atcaccaagc ctgccctcct ggtgtgtaag tatcagtgca tctgtctgcc ctgccagggg 540
tcttttcatg gacacccact atgccaggag cctccctggc ctgaagccag ccctgaagcc 600
ggctgccaca ctagcccaga gagaggagtg ccctgggagg gagatgggct gagtggagct 660
gtcatcaccc cctcctgacc tcgccttcaa ggtcaagttc tttggtgaga aggtcctagc 720
tgcattgcaa acagccaggt atagggattt gtgtttgtct gcgacccaga atcactgggg 780
ttcgagttag ggttcagatc tgagccaggt tagggggtta atgtcagggg gtaaagatta 840
ggaggttggt gtatatttgg tgttgggggt cactctatgg ccaaagtcag gggttgccat 900
gagctcaggt gacggaggct ccatcactga ctgtttgtga ctttgccagc tcccctggcc 960
ctctctgggc ctcagtctct tgctcatata ataagggtat agggaggcta aatgatacaa 1020
tttctaaaat agagtatcgc caagttcaaa agccagaatt atagacccca ggactacaga 1080
cagtgtcaca gcatcgtctg ggtgaggcta gggttagtgt gcggctgggc tcagggctgc 1140
cccatttgct aggatcgtgg ggttcccatg tgtcaggatc cagaggctag ggtatgatca 1200
ggatctctag ctggggtcag ggtcagagct ctctgtgtcc cctagaattg ccatcaacct 1260
taaacccaga ggaggcccag tccaacccct cagctttaag acctgggagc ctcatctcag 1320
agaggctgag tcatggccaa ggcagttggg gtgggagcag ggggcttggt gtgggcctgc 1380
agccctcatc cactgccctc cctctagtga accacgagac tgccaaggtg atccagaccg 1440
ccttccagcg agccagctac ccagatatca cgggcgagaa ggccatgatg ctccttggcc 1500
aagtcaagta tgggttgcac aagtgagtcg ggcctcgggt gtgacctggc tgggggtagg 1560
gtggcgggag gaacagcctg ggcttccccc agccacaggg aggaaaggca gcagctgggg 1620
gactcaggtc tctccccttg atttggaacc agagcc 1656

<210> 2
<211> 3446
<212> DNA
<213> Homo sapiens

<400> 2
ctctttttta aagataggca tttctagata taaatctccc tgtgagcacg gttccctcca 60
tcttcagcac accagggttg actctctccg ggcgttcttc cctggtcacc tctccccttc 120
ctctcctctt ctgcctcctc ttccactttt cggtaccctg tgattgattg ggaccaccca 180
gataacctag gatcatctcc ccacctaccc caaggtcctt aacttaacca tacttcatat 240
gggtaacacg agttgagtgt ggtacccagg tttgacatgt tgggtaacat atttgcaggt 300
tctgtggatt aggaggacat tttgggggcc atgattctat cttccaccct cgcctagaca 360
aaattggagg ctcactcctt gggctccctg gatgaccccc aacatccttc ctcacttcca 420
ttccttccca gcatccagat cagccacttg tccatcgcca gcagccaggt ggagctggtg 480
gaagccaagt ccattgatgt ctccattcag aacgtgtctg tggtcttcaa ggggaccctg 540
aagtatggct acaccactgc ctggtggtaa gcattcctgt cagctgatgc cccatgccct 600
ggccctctct gggtggaggg ctgaatgagg tctgggtcct tggctctttc caggctgggt 660
attgatcagt ccattgactt cgagatcgac tctgccattg acctccagat caacacacag 720
ctgagtatgt gtcaagcgtc ctctggggaa gtgggagctg gactccaggg cttggctcag 780
cagaggggga ggttgtgcag gcagagggtt ctggggccac caaaggaggc agcctgggaa 840
gtttgcaggg ttggggaccc cagagctggc caagctcttg actggcctgg gcagcatgtg 900
gataccatct gatagcggag gctgccctga ggtcatgtcg ggtctccctg cagcctgtga 960
ctctggtaga gtgcggaccg atgcccctga ctgctacctg tctttccata agctgctcct 1020
gcatctccaa ggggagcgag agtaagtaca ccaccctgtg cccccattcc tgtcgtgccc 1080
atcctgttag tgtgtccacg gccccctcca ggctcaaccc cacacaggga tgcttgtggg 1140
tggccaaacc tgagggcagc aataccttca gtggggtcat tccatccccc tccatcaata 1200
caccctaaag gctggaaaca acaataacca acagctagta actaacagct attaagaact 1260
tctgttggca aagcactatt ccaagccctt tcatgaatta attgattttg tccttaaaac 1320
caaccctagg atatagattc tgttatcatc ccctttttac atatgggtaa actgagtcac 1380
agacaggtta gaaaggaaaa gctcatatct acggagtcga tcctgcattc caagcaccac 1440
actaactcag agataaaact ctagccaagc taagtaactt gctgaggaca cacaactcgc 1500
cactaaggga tgggagtagg acccatttga acccagactt ctctgacccc agaagctgag 1560
ttcctagata ctttactctc ctgcttccca gggtggggct ttttgtcttg gccaacaccc 1620
tctgtcaagg agctgtggta accccattgc acagaggaag ataacaaggt ttggagagtc 1680
cctagtcatg ttaccaatgc caaacctgga aggcagaagg gaactggtgg gtggggtctg 1740
gagaggagcc ctctattcag gccatttttt ctgactctgg agcaagacgg atacatgtat 1800
gaatttggac tctagacacg ttctcgtgtg tgtgacaggt gtgagcgtca caggagctgg 1860
gccctcccga ggaattctgg atggtgccac agttaattct tgggtctgag gctccgtgtt 1920
ctcactgcaa aatgggagtg ataattctta cttcctgagc tacaagagtc agggccaaca 1980
gagccatgaa ggagcctggt acacactagg cgctccatgg atgcacagga ctggtcaggg 2040
gctcattgtg gtgcttgctg ccttcaggcc tgggtggatc aagcagctgt tcacaaattt 2100
catctccttc accctgaagc tggtcctgaa gggacaggtg agtgaggctg gctgactccc 2160
tgtggtccag gccatgccca ggaggctgga tccctttcct ccctgccttt ccctgagaag 2220
gtgccactcc caccttctcc atgtggccag tcccctgtgc cggtccccag cactgccacc 2280
accacgcagc tggaaggagg cactccgtct ggcctccttt cctgcctgga aagcacctgc 2340
tctgtctgcc ccagatctgc aaagagatca acgtcatctc taacatcatg gccgattttg 2400
tccagacaag ggctggtgag tgcgtttctg tctgcatgcc tcagaagaca gcagtgggag 2460
ccagaaagcc acctgctgca ctatgtggcc ttgggactgt cactcttcct gtctaggtcc 2520
catgggctct atctggctct gacacttgat gattagttat gagcatactt tggcaaagct 2580
ctgccccttt ggtgcggctc acaagctgtg tggcgaaggg cttgtctata gaactcagga 2640
caaatgggtg attaagtcca agaggcatcc aagattctcc tggaagtaga ttaggaaaaa 2700
agataattag attgctcaca tggctgggca ctcatccatg tactgtactc tcctatgcag 2760
tacagagcag agctgggttt cagcccaagt cttggactct gctctgaacc aaccttctag 2820
aagggctcta cctacccaga cagacagact tgggaaaaga gagaatgaaa aagtgccaca 2880
cccctccccg cacacccagg tcccacttta cagaggggaa cactgaggct ggagggttgg 2940
gtagctgtgt ggatgcaggg gacggtgact cagggcaatt cccccatccc tgaggccctg 3000
cgttgatctt ttcctcctgc agccagcatc ctttcagatg gagacattgg ggtggacatt 3060

tccctgacag gtgatcccgt catcacagcc tcctacctgg agtcccatca caaggtagga 3120
gttgtgggag ggtgggcagg gcccagcttc cccaggggag ttggtccttt tttgtgctct 3180
gacaacccca tcccccagct tcaaccttat ggcagccaag agtcctgggg agctcctcct 3240
cattcctgat gctcctccgc attcctgatg ctgcgaggag ggcaggccac agcgacgtgc 3300
ccctgacccc tctctgcagg caccagggct gcccactaca aggatcccag caaagcacca 3360
gctccttcct agagggctta ttcggcttct gtcatcctct acagcagtgg attgtggccc 3420
cccccagggg gtactgacaa aagctt 3446

<210> 3
<211> 1420
<212> DNA
<213> Homo sapiens

<400> 3
acatggtgca catgcctgta gtcctagcta cttggtggct gaggtagaca atcgcttgaa 60
cctgggacgt ggaggttgca gtgagctgag atcgtgccac tgccctccag cctgggcaac 120
agagtgagac tgtctcaaaa acaaaaaaag aaaagaaaag aaaaagaaag tgacttctca 180
ggtcctaacc ccaaagccac aggtgctggg gaactttcct cggttttcag aagagcagta 240
gctaagcctg gttcccgtgt catccttgcc tctccagtcc ctcagtggaa agaatcaggg 300
gccctgagct aggagggttg ctctctgctt cgggaagagc cctggctcac agcaaatttg 360
gtttctctcc ccaggatatc gtgactaccg tccaggcctc ctattctaag aaaaagctct 420
tcttaagcct cttggatttc cagtatgtgc tgcagagaag agaagggggc ggtcaactcc 480
gcaaacctct ccctggcccc ttggagtcag gcacagggcg gggtgttggt ggggaaatgt 540
ggcccctttc ttctggggca tatgggctga ctgcagggaa gataagaccc tgcctagata 600
gaatcttcgt ggggaagaag gggctccagg aagaatggag ggctgccagg aagaaggcct 660
gagctatgag acaaaagcac tggctgctat tcttagagtt tctttcccag gggatgttac 720
aggagggggc ccaatggagg gtcaaattat catcgctttt ttatttcagg attacaccaa 780
agactgtttc caacttgact gaggtaggta gtcttggata gactggggga aataagtcct 840
gtgggacctc ctgccttaaa gaaagcaggc ggagggccct aaaggaaatc aggcaaccag 900
accaaaagaa tgtgaccagg tggtccatgc tgtgtctctt gtgacccttc ttctccctgc 960
catgtctttt gggagagccc ttgtgttgca aaaatgagag tgtggtggta tggattgggg 1020
tttaggcaga acagtactgg ccaagcagcg ctccctggac ctcaattttc cctctgtgga 1080
atgggctagc aatcctgggc ctccccaggg cgaaggaaag accactcagg aagggcaccg 1140
tctggggcag gaaaacggag tgggttggat gtattttttt cacggatggg catgaggatg 1200
aatgcttgtc caggccgtgc agcatctgcc ttgtgggtca cttctgtgct ccagggagga 1260
ctcaccatgg gcatttgatt gcagagcagc tccgagtccg tccagagctt cctgcagtca 1320
atgatcaccg ctgtgggcat ccctgaggtc atgtctcgta agtgtgggct ggaggggaaa 1380
ctgggtgccg aggctgacag agcttcccat ttcacctttt 1420

<210> 4
<211> 894
<212> DNA
<213> Homo sapiens

<400> 4
ggatgggttg ggagctcaag ttttggggca gaagggaatt ttttttggca gcagagtgca 60
agccctgccg ccaggcaaac tctgctcttc ctcatcctca gaagcacttg ctcactctgc 120
taaatcaaag tgaaacgcat gtttacagaa tattggtcca aaagggtctc agcatctccc 180
actacccagg gtgcagagcc tcgggccggc cttgctcccc aagaagggct gactggggct 240
ctgtcccctc gcccagggct cgaggtagtg tttacagccc tcatgaacag caaaggcgtg 300
agcctcttcg acatcatcaa ccctgagatt atcactcgag atgtgagtac aaagcccccc 360
tcaccagccc ctgttcctgg ggagagaggc ccagacagga ttcctggggt gactgggggc 420
tgttggggag acagacagag gggcctctac cagcttggct ccctcctggt ggcctgggag 480
tcagcccagc tcgcccctct ctcctactgc ccctcccttc agggcttcct gctgctgcag 540
atggactttg gcttccctga gcacctgctg gtggatttcc tccagagctt gagctagaag 600

tctccaagga ggtcgggatg gggcttgtag cagaaggcaa gcaccaggct cacagctgga 660
accctggtgt ctcctccagc gtggtggaag ttgggttagg agtacggaga tggagattgg 720
ctcccaactc ctccctatcc taaaggccca ctggcattaa agtgctgtat ccaagagctg 780
cggagtcctt cttctgtggc tggcgggtag aggggggggg aagggattgt ctcaccagtg 840
ccgtccacct cttttcagcc cttccaagca gctgccccca aaccctccaa gctt 894

<210> 5
<211> 21
<212> DNA
<213> Homo sapiens

<400> 5
gttctttggt gagaaggtcc t 21

<210> 6
<211> 21
<212> DNA
<213> Homo sapiens

<400> 6
gttctttggt aagaaggtcc t 21

<210> 7
<211> 23
<212> DNA
<213> Homo sapiens

<400> 7
tggcctgaac ctgatcgcgg acc 23

<210> 8
<211> 23
<212> DNA
<213> Homo sapiens

<400> 8
tggcctgaac ttgatcgcgg acc 23

<210> 9
<211> 21
<212> DNA
<213> Homo sapiens

<400> 9
gatgatctag aggggcgggg g 21

<210> 10
<211> 21
<212> DNA
<213> Homo sapiens

<400> 10
gatgatctag tggggcgggg g                                                21


<210> 11
<211> 20
<212> DNA
<213> Homo sapiens

<400> 11
gaatggaggg agggcctggc                                                  20


<210> 12
<211> 35
<212> DNA
<213> Homo sapiens

<400> 12
gaatggaggg ctgccaggaa gaaggagggc ctggc                                 35


<210> 13
<211> 21
<212> DNA
<213> Homo sapiens

<400> 13
agcccagctc gcccctctct c                                                21


<210> 14
<211> 21
<212> DNA
<213> Homo sapiens

<400> 14
agcccagctc acccctctct c                                                21

## Description

### FIELD OF THE INVENTION

The present invention relates to technologies for assessing a subjects susceptibility to development of a cardiovascular disorder. The invention further relates to methods for selecting therapeutics which will be optimally effective for treating patients suffering from a cardiovascular disorder. The invention includes methods, compositions and kits useful for determining a patients cholesteryl ester transfer protein (CETP) genotype.

### BACKGROUND OF THE INVENTION

Atherosclerosis is one of the most widespread health problems in the United States today as are its attendant complications, particularly coronary heart disease. Atherosclerosis (or arteriosclerosis) is the term used to describe progressive luminal narrowing and hardening of the arteries that can result in an aneurysm, ischemia, thrombosis, embolism formation or other vascular insufficiency. The disease process can occur in any systemic artery in the human body. For example, atherosclerosis in the arteries that supply the brain (e.g., the carotids, intracerebral, etc.,) can result in stroke. Gangrene may occur when the peripheral arteries are blocked, and coronary artery disease occurs when the arteries that supply oxygen and nutrients to the myocardium are affected.

The atherosclerosis process involves lipid induced biological changes in the arterial walls resulting in a disruption of homeostatic mechanisms that keeps the fluid phase of the blood compartment separate from the vessel wall. In fact, the atheromatous plaque consists of a mixture of inflammatory and immune cells, fibrous tissue, and fatty material such as low density lipoproteins (LDL).

A number of risk factors have been associated with the development of premature atherosclerosis, primarily elevated plasma cholesterol levels. Due to the crucial role cholesterol appears to play in the occurrence of heart disease, a great deal of attention has been devoted to studying its synthesis, transport and metabolism in the human body.

Of particular interest is the establishment of relationships between the levels of plasma lipoproteins or serum lipids and the risk of development of coronary heart disease. Both high density lipoproteins (HDL) and low density lipoproteins (LDL) carry cholesterol mainly in the form of cholesteryl esters (CE). There are some indications, however, that while LDL cholesterol is a positive risk factor, HDL cholesterol is an even more important negative risk factor. Although the exact functions of these lipoproteins have not been completely established, HDL appears to serve for the removal of cholesterol from peripheral cells and its transport back to the liver, where a large proportion of the cholesterol excreted from the body is removed.

LDL and HDL are believed to play key roles in the development of cardiovascular disease by overloading the lysosomes of the walls of arterial cells with metabolites which are generally hydrolyzed slowly, such as CE and triglycerides. These products are evacuated from the liver and intestine by plasma LDL. When the amount of lipids to be transported exceeds the transporting capacity of HDL to the liver for excretion, CE become deposited in the cells in certain critical areas, such as arterial walls. This overloading eventually results in impaired cell function, and if continued may produce cell death. A continuous overloading results in the accumulation of cellular debris and the formation of atherosclerotic plaque in the vessel wall. This, in turn, leads to the blockage of the affected artery and/or muscular spasm, events which may manifest themselves as coronary heart disease or strokes. Thus, the level of HDL in plasma has been negatively correlated with the probability of developing atherosclerosis in humans and experimental animals.

Although the level of HDL has been shown to vary considerably among individuals, the means of regulation of such plasma level remains to be elucidated. In general, high levels of HDL have an anti-atherogenic effect whereas high levels of LDL have an atherogenic effect. The circulation in blood of compounds, such as cholesterol, that are insoluble in water requires the formation of particles. The insoluble components, e.g., cholesteryl esters and triglycerides, are packed in the core of the particles and surrounded by polar components such as proteins, phospholipids and the like. These particles are called lipoproteins, and have, thus, an outer polar shell and a non-polar core. These associations of lipoproteins containing CE in the core and, depending on their sizes and densities, have been named VLDL, IDL, LDL, and HDL.

VLDL is the largest lipoprotein secreted by the liver and is converted into IDL, and then to LDL, after the triglycerides contained by the VLDL are hydrolyzed by the lipoprotein lipase enzyme present on the surface of the arterial walls. LDL is the major lipoprotein that provides cholesteryl ester to extrahepatic and hepatic tissues. HDL is also secreted by the liver and is divided in HDL₂ and HDL₃ on the basis of size and density. A function of HDL is to pickup cholesterol from extrahepatic cells and deliver it to the liver, either through VLDL and LDL or through large HDL which is enriched with apo E. These large HDL particles are called HDL₁ and they do not stay in the plasma for long periods of time. They are rapidly removed by the liver or converted back to HDL₂ after donating their cholesteryl esters to VLDL and LDL.

Cholesteryl ester transfer protein (CETP) is a protein that may be isolated from the plasma of normal humans. CETP substrates include two neutrally charged or non-polar lipids, namely cholesteryl esters (CE) and triglycerides (TG). These neutral lipids are hydrophobic and present within the core of lipoprotein particles. They include, but are not limited to, high density lipoprotein (HDL), low density lipoprotein (LDL), intermediate density lipoprotein (IDL) and very low density lipoprotein (VLDL). Most lipoproteins are freely circulating in the plasma. The CETP transfers the two neutral lipids CE and TG from one lipoprotein particle, the donor, to another lipoprotein particle, the acceptor. A common donor-acceptor interaction is the transfer of the lipid CE from the lipoprotein HDL to the lipoprotein VLDL. Therefore, it has been suggested that CETP plays an important role in the regulation of plasma HDL levels.

Coronary artery disease is a multifactorial disease that results in the deposition of atheromatous plaque and progressive luminal narrowing of the arteries that supply the heart muscle. The luminal narrowing or blockage results in reduced ability to deliver oxygen and nutrients to the heart muscle, producing myocardial infarction, angina, unstable angina, and sudden ischemic death as heart failure. Though occlusion usually progresses slowly, blood supply may be cut off suddenly when a portion of the built-up arterial plaque breaks off and lodges somewhere in an artery to block it temporarily, or more usually, when thrombosis occurs within the arterial lumen. Depending on the volume of muscle distal to the blockage during such an attack, a portion of the myocardial tissue may die, weakening the heart muscle and often leading to the death of the individual.

For many years, the most common measure of eminent risk for a heart disease "clinical event", such as a myocardial infarction or death, was physical blockage of the coronary arteries, as assessed by techniques such as angiography. During the early 80's studies by DeWood and coworkers (*N. Engl. J. of Med*., (1980) 303:1137-40), revealed that occlusive thrombus was responsible for most cases of acute myocardial infarction. At that time, the prevailing concept was that myocardial infarction resulted from occlusion at a site of high grade stenosis. In 1988, Little et al. (*Circulation*, (1988) 78:1157-66), showed most of the infarctions resulted from a coronary occlusion that had previously shown a stenosis of less than 50% on angiography. Therefore, the severity of the coronary stenosis did not accurately predict the location of a subsequent coronary occlusion. With these studies the importance of vulnerable atherosclerotic plaque became evident.

The causes and mechanisms of the atheromatous plaque build-up are not completely understood, though many theories exist. One theory on the pathogenesis of atherosclerosis involves the following stages: (1) endothelial cell dysfunction and/or injury, (2) monocyte recruitment and macrophage formation, (3) lipid deposition and modification, (4) vascular smooth muscle cell proliferation, and (5) synthesis of extracellular matrix. According to this theory, the initiation of atherosclerosis is potentially due to a form of injury, possibly from mechanical stress or from chemical stress. How the body responds to this injury then defines whether, and how rapidly, the injury deteriorates into an atherosclerotic lesion. This, in turn, can result in arterial luminal narrowing and damage to the heart tissue which depends on the blood flow of oxygen and nutrients.

Though recent improvements in cardiovascular care have improved the life expectancy of coronary artery disease patients, this has been primarily from improvements in lowering lipid levels, limitation of damage after it has occurred, surgical restoration of blood supply, the suppression of abnormal heart rhythms and prevention of re-infarction. Little improvement has occurred, however, in early prevention of the disease by early diagnosis.

A key problem in treating coronary artery disease is proper diagnosis. Often the first sign of the disease is sudden death due to myocardial ischemia or myocardial infarction. Approximately half of all individuals who die of coronary artery disease die suddenly, Furthermore, for 40-60% of the patients who are eventually diagnosed as having coronary artery disease, myocardial infarction is the first presentation of the disease. Unfortunately, approximately 40% of those initial events go unnoticed by the patient. For various reasons, the perception of symptoms by the patient does not correlate well with the total burden of coronary artery disease (Anderson & Kin, Am. *Heart J.,* (1992) 123(5):1312-23).

Epidemiologic studies have indicated that an individual's genetic composition is a significant risk factor for development of a vascular disease. For example, a family history of heart disease is associated with an increased individual risk of developing coronary artery disease. Lipid and cholesterol metabolism have historically been considered the primary genetic influence on coronary artery disease. For example, deficiency in cell receptors for low-density lipids (LDL), such as in familial hypercholesterolemia, is associated with high levels of plasma LDL and premature development of atherosclerosis (Brown & Goldstein, Science, (1976) 191 (4223):150-4).

While the causes of atherosclerosis remain unknown, the proper diagnosis of susceptibility may provide patients sufficient time to reduce their risk of developing coronary artery disease. One method to reduce the risk of coronary artery disease is through alteration of patient lifestyle such as smoking cessation, exercise, weight loss, and stress reduction. Other methods include pharmaceutical intervention to treat hypertension, hypercholesterolemia, and diabetes, as well as the use of aspirin. Finally, genetic therapy promises to treat those rare genetic traits that result in a family history of cardiovascular disease (e.g., altered apolipoprotein metabolism).

The ability to identify high-risk individuals would allow physicians to focus preventive measures on those individuals who may gain the greatest benefit, and would provide strong incentives for those at risk to comply with such approaches.

CETP, the cholesteryl ester transferase protein, is a critical enzyme in cholesterol trafficking. CETP catalyzes the transfer of cholesteryl esters from HDL to LDL, VLDL and IDL in exchange for triglycerides. CETP therefore removes cholesterol from the retrograde pathway and returns cholesterol to LDL, which can deliver cholesterol to tissues throughout the body. Deficiencies in CETP activity are associated with increased HDL concentration, and high HDL levels are associated with a decreased risk of developing cardiovascular disorders.

The CETP gene is located on chromosome 16 (16q12-16q21) near the lecithin cholesterol acyl transferase (LCAT) gene. The CETP protein consists of 476 amino acids and is encoded for by the CETP gene which is over 25 kb in length and consists of 16 exons and 15 introns (Agellon et al. (1990) *Biochemistry* 29: 1372-1376).

It is understood that there may be a relation between the effects of certain risk factors on a patient afflicted with one of the abovementioned cardiovascular disorders and the development of the related disease, and there may be a relation between the effects of risk factors and the progression of the related disease. Diagnosis of the underlying haplotype pattern can guide the clinician in making recommendations or in designing interventions to decrease the impact of the risk factors on the particular disorder or disease. Furthermore, genetic effects on cardiovascular disease are likely to be multifactorial. Therefore, the determination of CETP genotypes in the context of large scale genotype screening of a patient may provide a highly informative data set for directing patient management.

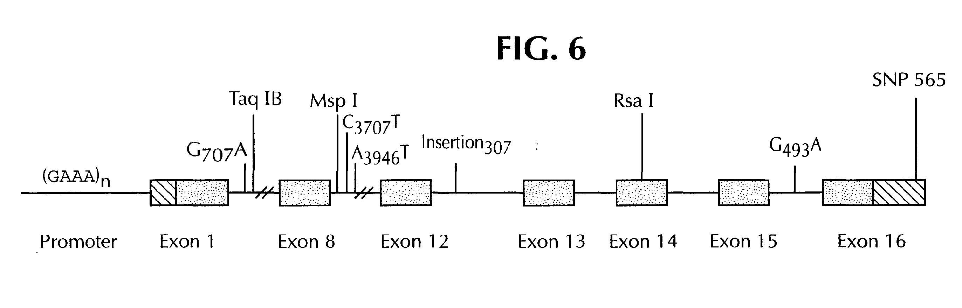

Several CETP polymorphisms have been identified in the literature. The Taq 1, Msp 1 and Rsa 1 polymorphisms (Figure 6) have been shown to be associated with modifications in CETP and HDL levels. Specifically, a B2 allele at Taq I (G → A mutation in intron I) (see e.g., Kuivenhoven et al., New Eng. J. Med. 338:86-93 (1998); Dullaart et al., Diabetes 46: 2082-2087 (1997); Kuivenhoven et al., Arteriosclerosis, Thrombosis & Vasc. Biol. 17: 560-8 (1997); Juvonen et al., J. Lipid Res. 36:804-12 (1995); Hannuksela et al., Atherosclerosis 110: 35-44 (1994); Bernard et al., J. Lipid Res. 39: 59-65 (1998); Vohl et al., Int. J. Obesity & Related Metab. Disorders 23: 918-25 (1999); Durlach et al., J. Clin. Endo. & Metab. 84: 3656-9 (1999); and Gudnason et al., Eur. J. Clin. Invest. 29: 116-128 (1999)); an M2 allele at Msp I (A → G mutation in intron 8) (see e.g., Kuivenhoven et al., Arteriosclerosis, Thrombosis & Vasc. Biol. 17: 560-8 (1997)); and an R1 allele at Rsa I (G → A mutation in exon 14 resulting in an amino acid substitution 1405V) (see e.g., Kakko et al., Eur. J. Clin. Invest. 30: 18-25 (2000); Kuivenhoven et al., Arteriosclerosis, Thrombosis & Vasc. Biol. 17: 560-8 (1997); Bruce et al., J. Lipid Res. 39: 1071-1078 (1998); and Gudnason et al., Eur. J. Clin. Invest. 29: 116-128 (1999)); have been associated with higher HDL levels and lower CETP mass.

The promoter of CETP contains a highly polymorphic region, referred to as the VNTR (Variable number of Tandem Repeats) polymorphism, which consists of varying numbers of GAAA tetranucleotide repeats between approximately nucleotides -2145 and -1940 (Williams et al., Gene 197: 101-107 (1997) and Talmud et al., *Circulation* 101: 2461-2466 (2000)). Genotype analysis of 119 twin pairs revealed 28 different alleles of CETP with fragment sizes ranging from 324 to 464 base pairs, with 384 bp being the most common allele (Talmud et al., *Circulation* 101: 2461-2466 (2000)). The 384 bp fragment contained 8 repeats of the GAAA tetranucleotide. Alleles containing from 324 to 400 bp were referred to as 'short alleles' and alleles containing 404 to 464 bp were referred to as 'long alleles'. The short alleles were shown to be in strong linkage disequilibrium with the Taq IB polymorphism and, thus, correlated with decreased CETP activity and mass.

The ability to rapidly genotype patients promises to radically change the testing and development of therapeutic or disease-preventative substances. Currently, the effectiveness of a substance for treating or preventing a disease is assessed by testing it on a pool of patients. Many variables in the patient pool are controlled for, but the effects of genetic variability are not typically assessed. A drug may be statistically ineffective when examined in a diverse pool of patients and yet be highly effective for a select group of patients with particular genetic characteristics. Unless patients are separated by genotype, many drugs with great promise for selected populations are likely to be rejected as useless for the population as a whole. If a patient pool can be segregated into groups based on genotype, drugs can be re-tested for their ability to affect genetically defined subgroups of patients. This type of screening may allow the resurrection of failed compounds, the identification of new compounds and the identification of new uses for well-known compounds.

Genetic screening (also called genotyping or molecular screening), can be broadly defined as testing to determine if a patient has mutations (or alleles or polymorphisms) that either cause a disease state, contribute to a disease state (i.e., are a risk factor associated with a disease state), are "linked" to the mutation causing a disease state, or are "linked" to the mutation which contributes to the disease state. Linkage refers to the phenomenon wherein DNA sequences that are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population. Indeed, where multiple polymorphisms in a given chromosomal region are found to be in linkage disequilibrium with one another, they define a quasi-stable genetic "haplotype." In contrast, recombination events occurring between two polymorphic loci cause them to become separated onto distinct homologous chromosomes. If meiotic recombination between two physically linked polymorphisms occurs frequently enough, the two polymorphisms will appear to segregate independently and are said to be in linkage equilibrium.

While the frequency of meiotic recombination between two markers is generally proportional to the physical distance between them on the chromosome, the occurrence of "hot spots" as well as regions of repressed chromosomal recombination can result in discrepancies between the physical and recombinational distance between two markers. Thus, in certain chromosomal regions, multiple polymorphic loci spanning a broad chromosomal domain may be in linkage disequilibrium with one another, and thereby define a broad-spanning genetic haplotype. Furthermore, where a disease-causing mutation is found within or in linkage with this haplotype, one or more polymorphic alleles of the haplotype can be used as a diagnostic or prognostic indicator of the likelihood of developing the disease. This association between otherwise benign polymorphisms and a disease-causing polymorphism occurs if the disease mutation arose in the recent past, so that sufficient time has not elapsed for equilibrium to be achieved through recombination events. Therefore identification of a human haplotype that spans or is linked to a disease-causing mutational change, serves as a predictive measure of an individual's likelihood of having inherited that disease-causing mutation. Importantly, such prognostic or diagnostic procedures can be utilized without necessitating the identification and isolation of the actual disease-causing lesion. This is significant because the precise determination of the molecular defect involved in a disease process can be difficult and laborious, especially in the case of multifactorial diseases such as coronary artery disease.

Indeed, the statistical correlation between a disease state and a polymorphism does not necessarily indicate that the polymorphism directly causes the disorder. Rather the correlated polymorphism may be a benign allelic variant which is linked to (i.e., in linkage disequilibrium with) a disorder-causing mutation which has occurred in the recent human evolutionary past, so that sufficient time has not elapsed for equilibrium to be achieved through recombination events in the intervening chromosomal segment. Thus, for the purposes of diagnostic and prognostic assays for a particular disease, detection of a polymorphic allele associated with that disease can be utilized without consideration of whether the polymorphism is directly involved in the etiology of the disease. Furthermore, where a given benign polymorphic locus is in linkage disequilibrium with an apparent disease-causing polymorphic locus, still other polymorphic loci which are in linkage disequilibrium with the benign polymorphic locus are also likely to be in linkage disequilibrium with the disease-causing polymorphic locus. Thus, these other polymorphic loci will also be prognostic or diagnostic of the likelihood of having inherited the disease-causing polymorphic locus. Indeed, a broad-spanning human haplotype (describing the typical pattern of co-inheritance of alleles of a set of linked polymorphic markers) can be targeted for diagnostic purposes once an association has been drawn between a particular disease or condition and a corresponding human haplotype. Thus, the determination of an individual's likelihood for developing a particular disease or condition can be made by characterizing one or more disease-associated polymorphic alleles (or even one or more disease-associated haplotypes) without necessarily determining or characterizing the causative genetic variation.

### SUMMARY OF THE INVENTION

This invention provides methods, compositions and kits, which are based at least in part upon the discovery of novel alleles of the CETP gene.

In a first aspect, the invention provides methods for determining a subject's predisposition to cardiovascular disease comprising detecting in a nucleic acid sample from said subject one or more alleles at one or more of the following CETP loci: intron 1 (707); intron 8 (3707); intron 8 (3946); promoter (VNTR); insertion (307); intron 15 (493), wherein detection of an allele indicates a subject's predisposition to cardiovascular disease.

Another aspect of the invention provides isolated nucleic acids comprising CETP alleles. In one embodiment, the nucleic acids comprise at least 11 consecutive nucleotides of SEQ. ID. Nos: 6, 8, 10, 12 or 14 or a complement thereof. In another embodiment, the nucleic acids comprise at least one GAAA repeat, or complement thereof, wherein said nucleic acid is amplified from the CETP promoter region corresponding to -2144 to -1974 nucleotides from the transcriptional start site.

Yet another aspect of the invention provides kits comprising a means for detecting one or more alleles at a CETP locus. In a preferred embodiment, the CETP alleles are selected from the group consisting of: intron 1 (707); intron 8 (3707); intron 8 (3946); promoter (VNTR); insertion (307); and intron 15 (493), and at least one primer oligonucleotide that hybridizes 5' or 3' to one of said CETP loci. In a preferred embodiment, the kit comprises a second oligonucleotide that hybridizes 5' or 3' to one of said CETP loci, such that the oligonucleotide pair can be used to amplify a region of between about 50 and about 1000 base pairs therebetween.

Still another aspect of the invention provides methods for treating a patient comprising detecting at least one CETP allele in a nucleic acid sample from said patient, diagnosing a cardiovascular disorder, selecting a cardiovascular disorder therapeutic, and providing the cardiovascular disorder therapeutic to the patient.

Another aspect of the invention provides methods for identifying a cardiovascular disorder therapeutic comprising contacting a subject carrying a CETP allele at one of the following loci: intron 1 (707); intron 8 (3707); intron 8 (3946); promoter (VNTR); insertion (307); and intron 15 (493); or a functional mutation that is in linkage disequilibrium with any of the above; with a test substance, and determining the effect of said test substance on CETP activity.

### BRIEF DESCRIPTION OF THE DRAWING

The foregoing and other objects of the present invention, the various features thereof, as well as the invention itself may be more fully understood from the following description, when read together with the accompanying figures, where:
FIGURE 1 shows the GenBank sequence (M32992; SEQ. ID. No:1) for a portion of the human CETP gene corresponding to exons 1 and 2;
FIGURE 2 shows the GenBank sequence (M32993; SEQ. ID. No:2) for a portion of the human CETP gene corresponding to exons 3-8;
FIGURE 3 shows the GenBank sequence (M32997; SEQ. ID. No:3) for a portion of the human CETP gene corresponding to exons 12-14;
FIGURE 4 shows the GenBank sequence (M32998; SEQ. ID. No:4) for a portion of the human CETP gene corresponding to exons 15 and 16;
FIGURE 5 lists alleles of the CETP gene;
FIGURE 6 shows a map of the CETP gene which indicates the location of the CETP polymorphisms of the invention ((GAAA)ₙ, G₇₀₇A, C₃₇₀₇T, A₃₉₆₄T, Insertion₃₀₇, G₄₉₃A) in relation to several polymorphisms from the literature (Taq IB, MSP I and RSA I);
FIGURE 7 shows a graph of the CETP concentration (µg/ml) in individuals with the CETP-Taq I polymorphism as compared to baseline. B1/B1 represents normal individuals, B1/B2 represents individuals that are heterozygous for the Taq IB mutant allele and B2/B2 represents individuals that are homozygous for the mutant Taq IB allele. The open circles represent the CETP concentration for individuals with the specified Taq IB genotype; the solid squares represent the mean CETP concentration and standard error (bar through square) for individuals with the specified Taq IB genotype;
FIGURE 8 shows a graph of the CETP concentration (µg/ml) in individuals with the CETP-Msp I polymorphism as compared to baseline. M1/M2 represents individuals that are heterozygous for the Msp I polymorphic allele and M2/M2 represents individuals homozygous for the mutant Msp I allele. The open circles represent the CETP concentration for individuals with the specified Msp I genotype; the solid squares represent the mean CETP concentration and standard error (bar through square) for individuals with the specified Msp I genotype;
FIGURE 9 shows a graph of the HDL concentration (mg/dl) in individuals with the SNP 565 polymorphism as compared to baseline. G/C represents individuals heterozygous for the SNP 565 mutant allele and G/G represents normal individuals. The open circles represent the HDL concentration for individuals with the specified SNP 565 genotype; the solid squares represent the mean HDL concentration and standard error (bar through square) for individuals with the specified SNP 565 genotype;

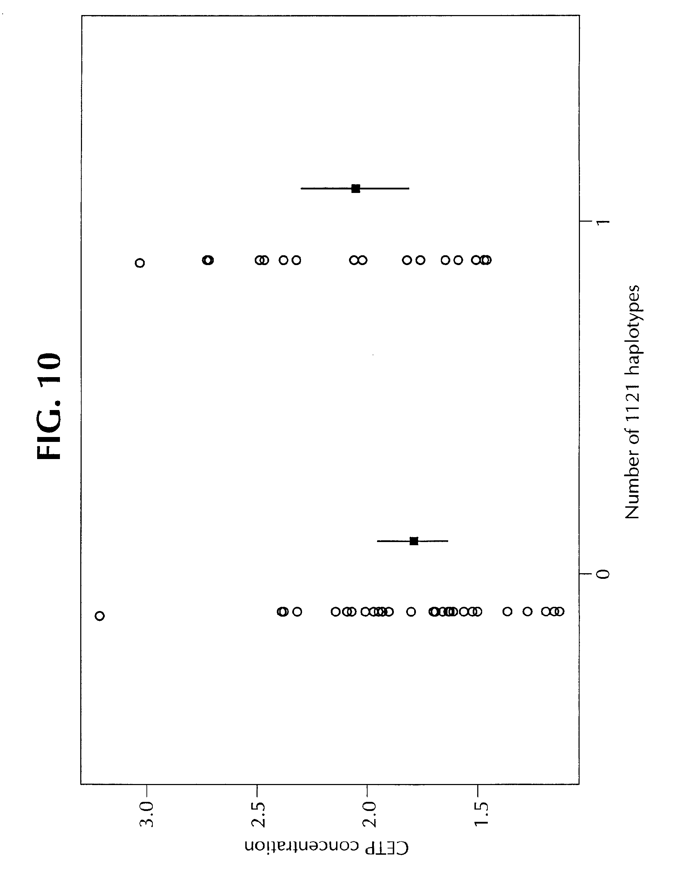

FIGURE 10 shows a graph of the CETP concentration (µg/ml) for individuals with 0 or 1 copy of the 1121 haplotype as compared to baseline. The 1121 haplotype represents individuals with an allele that is normal at each of the Taq I, Msp I, Rsa I and SNP 565 polymorphic sites. 0 represents individuals that are homozygous for an allele containing mutations at each of the four polymorphic sites and 1 represents individuals that are heterozygous for an allele containing mutations at each of the four polymorphic sites. The open circles represent the CETP concentration for individuals with the specified haplotype; the solid squares represent the mean CETP concentration and standard error (bar through square) for individuals with the specified haplotype; and

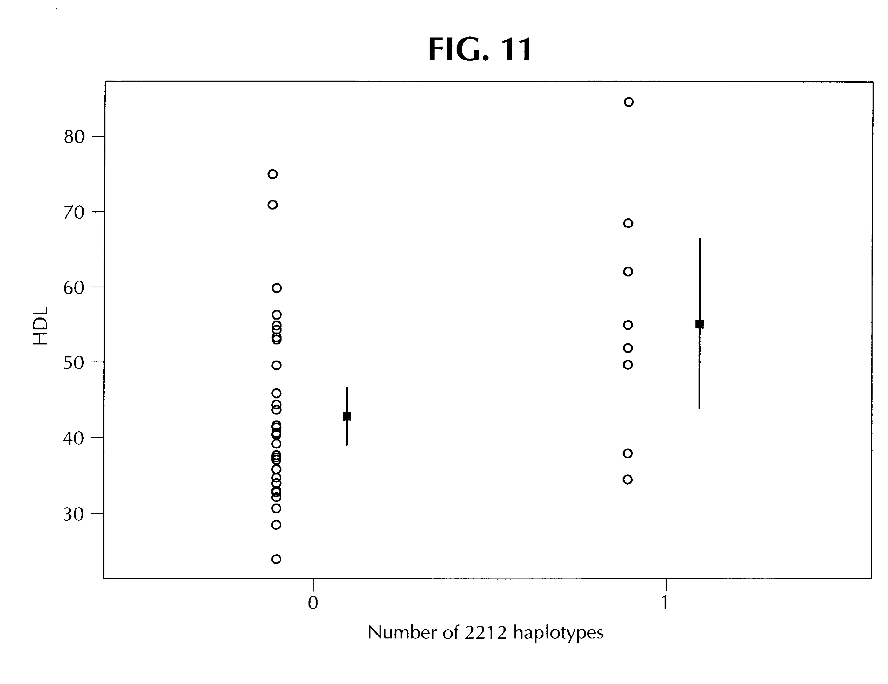

FIGURE 11 shows a graph of the HDL concentration (mg/dl) for individuals with 0 or 1 copy of the 2212 haplotype as compared to baseline. The 2212 haplotype represents individuals with an allele that is mutant at the Taq I, Msp I, Rsa I and SNP 565 polymorphic sites. 0 represents individuals that are homozygous for an allele that is normal at each of the polymorphic sites and 1 represents individuals that are heterozygous for an allele containing mutations at each of the four polymorphic sites. The open circles represent the HDL concentration for individuals with the specified haplotype; the solid squares represent the mean HDL concentration and standard error (bar through square) for individuals with the specified haplotype.

### DETAILED DESCRIPTION OF THE INVENTION

Those skilled in the art will fully understand the terms used herein to describe the present invention; nonetheless, for convenience, the following terms used herein, including the appendant claims, unless otherwise provided herein, are as described below:
"aberrant activity", as applied to an activity of a polypeptide such as CETP, refers to an activity which differs from the activity of a polypeptide encoded by the wild-type or most common allele or which differs from the activity of the polypeptide in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in an activity. For example an aberrant polypeptide can have an altered substrate specificity. A cell can have an aberrant CETP activity due to overexpression or underexpression of a gene encoding CETP;
"allele" refers to the different sequence variants found at different polymorphic regions. For example, CETP intron (707) has at least two different alleles. The sequence variants may be single or multiple base changes, including without limitation insertions, deletions, or substitutions, or may be a variable number of sequence repeats;
"amplification" as used herein in reference to nucleic acids is intended to encompass essentially any method of generating many copies of a nucleic acid, either in single or double stranded form. Such methods include but are not limited to polymerase chain reaction (PCR) and replication of the nucleic acid in cells;
"antibody " as used herein is intended to refer to a binding agent including a whole antibody or a binding fragment thereof which is specifically reactive with a CETP polypeptide. Antibodies can be fragmented using conventional techniques and the fragments screened for utility in the same manner as described above for whole antibodies. For example, F(ab)2 fragments can be generated by treating an antibody with pepsin. The resulting F(ab)2 fragment can be treated to reduce disulfide bridges to produce Fab fragments. The antibody of the present invention is further intended to include bispecific, single-chain, and chimeric and humanized molecules having affinity for a CETP polypeptide conferred by at least one CDR region of the antibody;
"bioactive portion of CETP" refers to a fragment of a full-length CETP, wherein the fragment specifically mimics or antagonizes at least one activity of a wild-type CETP;
"biological activity" or "bioactivity" or "activity" or "biological function", which are used interchangeably, for the purposes herein when applied to CETP means an effector or antigenic function that is directly or indirectly performed by a CETP (whether in its native or denatured conformation), or by any subsequence (fragment) thereof. A biological activity can include binding substrate, causing the transfer of lipids, effecting signal transduction from a receptor, modulation of gene expression or an antigenic effector function;
"cardiovascular disease" is a cardiovascular disorder, as defined herein, characterized by clinical events, clinical symptoms and/or clinical or subclinical signs. Clinical symptoms are those experiences reported by a patient that indicate to the clinician the presence of pathology. Clinical signs are those objective findings on physical or laboratory examination that indicate to the clinician the presence of pathology. "Cardiovascular disease" includes both "coronary artery disease" and "peripheral vascular disease," both terms being defined below. Clinical symptoms in cardiovascular disease include chest pain, shortness of breath, weakness, fainting spells, alterations in consciousness, extremity pain, paroxysmal nocturnal dyspnea, transient ischemic attacks and other such phenomena experienced by the patient. Clinical signs in cardiovascular disease include such findings as EKG abnormalities, altered peripheral pulses, arterial bruits, abnormal heart sounds, rales and wheezes, jugular venous distention, neurological alterations and other such findings discerned by the clinician. Clinical symptoms and clinical signs can combine in a cardiovascular disease such as a myocardial infarction (MI) or a stroke (also termed a "cerebrovascular accident" or "CVA"), where the patient will report certain phenomena (symptoms) and the clinician will perceive other phenomena (signs) all indicative of an underlying pathology. A cardiovascular disease resulting from an occlusive disorder can be termed an "occlusive disease." Clinical events associated with occlusive disease include those signs and symptoms where the progressive occlusion of an artery affects the amount of circulation that reaches a target tissue. Progressive arterial occlusion may result in progressive ischemia that may ultimately progress to tissue death if the amount of circulation is insufficient to maintain the tissues. Signs and symptoms of occlusive disease include claudication, rest pain, angina, and gangrene, as well as physical and laboratory findings indicative of vessel stenosis and decreased distal perfusion. As yet another example, a cardiovascular disease resulting from restenosis can be termed an in-stent stenosis disease. Instent stenosis disease includes the signs and symptoms resulting from the progressive blockage of an arterial stent that has been positioned as part of a procedure like a percutaneous transluminal angioplasty, where the presence of the stent is intended to help hold the vessel in its newly expanded configuration. The clinical events that accompany in- stent stenosis disease are those attributable to the restenosis of the reconstructed artery;
"cardiovascular disorder" refers broadly to both to coronary artery disorders and peripheral arterial disorders. The term "cardiovascular disorder" can apply to any abnormality of an artery, whether structural, histological, biochemical or any other abnormality. A "cardiovascular disorder" can occur in an artery primarily, that is, prior to any medical or surgical intervention. Primary cardiovascular disorders include, among others, atherosclerosis, arterial occlusion, aneurysm formation and thrombosis. A "cardiovascular disorder" can occur in an artery secondarily, that is, following a medical or surgical intervention. Secondary cardiovascular disorders include, among others, post-traumatic aneurysm formation, restenosis, and postoperative graft occlusion;
"cardiovascular disorder causative functional mutation" refers to a mutation which causes or contributes to the development of a cardiovascular disorder in a subject. Preferred mutations occur within the CETP gene. A cardiovascular disorder causative functional mutation occurring within the CETP gene may alter, for example, the open reading frame or splicing pattern of the gene, thereby resulting in the formation of a hyperactive gene product;
"cardiovascular disorder therapeutic" refers to any agent or therapeutic regimen (e.g., pharmaceuticals or surgical means) that prevents or postpones the development of or reduces the extent of an abnormality constitutive of a cardiovascular disorder in a subject. Cardiovascular disorder therapeutics can be directed to the treatment of any cardiovascular disorder. Examples of therapeutic agents directed to each category of cardiovascular disorder are provided herein. It is understood that a therapeutic agent may be useful for more than one category of cardiovascular disorder. The therapeutic can be a polypeptide, peptidomimetic, nucleic acid or other inorganic or organic molecule, preferably a "small molecule". Preferably the therapeutic can modulate at least one activity of a CETP polypeptide, e.g., transfer of cholesteryl esters, by mimicking or potentiating (agonizing) or inhibiting (antagonizing) the effects of a naturally- occurring polypeptide. A CETP agonist can be a wild-type protein or derivative thereof having at least one bioactivity of the wild-type. A CETP agonist can also be a compound that upregulates expression of a gene or which increases at least one bioactivity of a protein. CETP agonist can also be a compound which increases the interaction of a polypeptide with another molecule, e.g., a cholesteryl ester. A CETP antagonist can be a compound that inhibits or decreases CETP activity, for example by inhibiting CETP production, or preventing CETP from interacting with the appropriate substrates. A CETP antagonist can be a compound that downregulates expression of a gene or which reduces the amount of a protein present. The antagonist can be a dominant negative form of a polypeptide, e.g., a form of a polypeptide which is capable of interacting with a target peptide, e.g., a molecule that targets CETP to the appropriate lipoprotein complexes, but which does not promote the activation of the receptor. The antagonist can also be a nucleic acid encoding a dominant negative form of a polypeptide, an antisense nucleic acid, or a ribozyme capable of interacting specifically with an RNA. Yet other antagonists are molecules which bind to a polypeptide and inhibit its action. Such molecules include peptides, e.g., forms of target peptides which do not have biological activity, and which inhibit binding to receptors. Thus, such peptides will bind to the active site of a protein and prevent it from interacting with target peptides. Yet other antagonists include antibodies that specifically interact with an epitope of a molecule, such that binding interferes with the biological function of the polypeptide. In yet another preferred embodiment, the antagonist is a small molecule, such as a molecule capable of inhibiting the interaction between a polypeptide and a target receptor. Alternatively, the small molecule can function as an antagonist by interacting with sites other than the receptor binding site;
"cells", "host cells" or "recombinant host cells" are terms used interchangeably herein to refer not only to the particular subject cell, but to the progeny or potential progeny of such a cell. Because certain modifications may occur in succeeding generations due to either mutation or environmental influences, such progeny may not, in fact be identical to the parent cell, but is still included within the scope of the term as used herein;
"cerebrovascular disease," as used herein, is a type of peripheral vascular disease (as defined below) where the peripheral vessel blocked is part of the cerebral circulation. The cerebral circulation includes the carotid and the vertebral arterial systems. This definition of cerebrovascular disease is intended specifically to include intracranial hemorrhage that does not occur as a manifestation of an arterial blockage. Blockage can occur suddenly, by mechanisms such as plaque rupture or embolization. Blockage can occur progressively, with narrowing of the artery via myointimal hyperplasia and plaque formation. Blockage can be complete or partial. Certain degrees and durations of blockage result in cerebral ischemia, a reduction of blood flow that lasts for several seconds to minutes. The prolongation of cerebral ischemia can result in cerebral infarction. Ischemia and infarction can be focal or widespread. Cerebral ischemia or infarction can result in the abrupt onset of a non-convulsive focal neurological defect, a clinical event termed a "stroke" or a "cerebrovascular accident (CVA)". Cerebrovascular disease has two broad categories of pathologies: thrombosis and embolism. Thrombotic strokes occur without warning symptoms in 80-90% of patients; between 10 and 20% of thrombotic strokes are heralded by transient ischemic attacks. A cerebrovascular disease can be associated with a fragile plaque disorder. The signs and symptoms of this type of cerebrovascular disease are those associated with fragile plaque, including stroke due to sudden arterial blockage with thrombus or embolus formation. A cerebrovascular disease can be associated with occlusive disorder. The signs and symptoms of this type of cerebrovascular disease relate to progressive blockage of blood flow with global or local cerebral ischemia. In this setting, neurological changes can be seen, including stroke;
"CETP" refers to the cholesteryl ester transferase protein. CETP is an enzyme that mediates the transfer of cholesteryl esters from HDL to VLDL, IDL or LDL in exchange for triglycerides. "CETP" may also be used in reference to the gene encoding CETP, and may be referred to as "CETP gene" as necessary;
"CETP agonist" as used herein refers to an agent that mimics, upregulates (potentiates or supplements) or otherwise increases a CETP bioactivity. CETP agonists may act on any of a variety of different levels, including regulation of CETP gene expression, regulation of mRNA splicing mechanisms, stabilization of mRNA, phosphorylation of proteins for translation, maturation and secretion of CETP, or by affecting the biochemical activities of CETP;
"CETP antagonist" as used herein refers to an agent that downregulates or otherwise decreases a CETP bioactivity. CETP agonists may act on any of a variety of different levels, including regulation of CETP gene expression, regulation of mRNA splicing mechanisms, stabilization of mRNA, phosphorylation of proteins for translation, maturation and secretion of CETP, or by affecting the biochemical activities of CETP;
"CETP loci" as used herein include all the nucleic add sequence at or near the CETP gene, introns, exons and 5' and 3' untranslated regions. The GenBank Accession Nos. for the CETP gene include M32992, M32993, M32997 and M32998;
"CETP functional mutation" refers to a mutation within or near the CETP gene that results in an altered phenotype.
"CETP X (Z) allele Y" refers to a particular allelic form, designated Y, occurring at a CETP polymorphic site in region X, wherein X is an intron, exon or 5' or 3' untranslated region and positioned at or near nucleotide Z, wherein nucleotide Z is numbered relative to the appropriate GenBank sequence (except in the case of the locus: CETP promoter (VNTR -2144 - -1974) for which the numbering is relative to the major translation start site) (see Table 1). In addition, if the mutation is an insertion or variable nucleotide tandem repeat (VNTR) this information is also indicated in the parentheses. As most of the alleles herein are alleles of CETP, the term "CETP" is often omitted. For example, intron 1 (707) refers to the locus at 707 When a subject has two identical CETP alleles, the subject is said to be homozygous, or to have the homozygous state. When a subject has two different CETP alleles, the subject is said to be heterozygous, or to have the heterozygous state;
"CETP polypeptide" and "CETP protein" are intended to encompass polypeptides comprising the amino acid sequence encoded by the CETP genomic DNA sequences or fragments thereof, and homologs thereof and include agonist and antagonist polypeptides;
"chimera," "mosaic," "chimeric mammal" and the like, refers to a transgenic mammal with a knock-out or knock-in construct in at least some of its genome-containing cells;
"clinical event" is an occurrence of clinically discernible signs of a disease or of clinically reportable symptoms of a disease. "Clinically discernible" indicates that the sign can be appreciated by a health care provider. "Clinically reportable" indicates that the symptom is the type of phenomenon that can be described to a health care provider. A clinical event may comprise clinically reportable symptoms even if the particular patient cannot himself or herself report them, as long as these are the types of phenomena that are generally capable of description by a patient to a health care provider;
"control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the CETP gene. However, where the sample to be tested is genomic DNA, the control sample is preferably a highly purified sample of genomic DNA.
"coronary artery disease" ("CAD") refers to a vascular disorder relating to the blockage of arteries serving the heart. Blockage can occur suddenly, by mechanisms such as plaque rupture or embolization. Blockage can occur progressively, with narrowing of the artery via myointimal hyperplasia and plaque formation. Those clinical signs and symptoms resulting from the blockage of arteries serving the heart are manifestations of coronary artery disease. Manifestations of coronary artery disease include angina, ischemia, myocardial infarction, cardiomyopathy, congestive heart failure, arrhythmias and aneurysm formation. It is understood that fragile plaque disease in the coronary circulation is associated with arterial thrombosis or distal embolization that manifests itself as a myocardial infarction. It is understood that occlusive disease in the coronary circulation is associated with arterial stenosis accompanied by anginal symptoms, a condition commonly treated with pharmacological interventions and with angioplasty;
"disorder associated allele" or "an allele associated with a disorder" refers to an allele whose presence in a subject indicates that the subject has or has an increased propensity for developing a particular disorder. One type of disorder associated allele is a "cardiovascular disorder associated allele," the presence of which in a subject indicates that the subject has or is susceptible to developing a cardiovascular disorder. A number of phenotypic indicators are predictive of cardiovascular disease risk, including high cholesterol and LDL as well as low HDL. A "cardiovascular disorder associated allele" may also be associated with these predictive phenotypic markers. Examples of alleles associated with lower HDL (high CETP concentration) include CETP (TaqI) allele 1; CETP (Mspl) allele 1; CETP (Rsal) allele 2; CETP (snp565) allele 1 and CETP Insertion (307) wt;
"disruption of the gene" and "targeted disruption" or any similar phrase refers to the site specific interruption of a DNA sequence so as to prevent expression of that gene in the cell as compared to the non-disrupted copy of the gene. The interruption may be caused by deletions, insertions or modifications to the gene, or any combination thereof;
"embolus," "embolism" or "embolization" refer to artery- to-artery embolism or embolization;
"haplotype" as used herein is intended to refer to a set of alleles that are inherited together as a group (are in linkage disequilibrium) at statistically significant levels (pcorr < 0.05). As used herein, the phrase "a CETP haplotype" refers to a haplotype including CETP loci;
"HDL" means high-density lipoprotein;
"increased risk" refers to a higher frequency of occurrence of the disease or disorder in an individual in comparison to the frequency of occurrence of the disease or disorder in a population. A factor identified to be associated with increased risk is termed a "risk factor." Carrying a particular polymorphic allele is a risk factor for a particular cardiovascular disease, and is associated with an increased risk of the particular disease.
"interact" as used herein is meant to include detectable relationships or associations (e.g., biochemical interactions) between molecules, such as interactions between protein-protein, protein-nucleic acid, nucleic acid-nucleic acid and protein-small molecule or nucleic acid-small molecule in nature;
"isolated" as used herein with respect to nucleic acids, such as DNA or RNA, refers to molecules separated from other DNAs, or RNAs, respectively, that are present in the natural source of the macromolecule. For example, an isolated nucleic acid encoding CETP preferably includes no more than 10 kilobases (kb) of nucleic acid sequence which naturally immediately flanks the CETP gene in genomic DNA, more preferably no more than 5 kb of such naturally occurring flanking sequences, and most preferably less than 1.5 kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides;
"knock-in" transgenic animal refers to an animal that has had a modified gene introduced into its genome and the modified gene can be of exogenous or endogenous origin;
"knock-out" transgenic animal refers to an animal in which there is partial or complete suppression of the expression of an endogenous gene (e.g., based on deletion of at least a portion of the gene, replacement of at least a portion of the gene with a second sequence, introduction of stop codons, the mutation of bases encoding critical amino acids, or the removal of an intron junction, etc.);
"knock-out construct" refers to a nucleic acid sequence that can be used to decrease or suppress expression of a protein encoded by endogenous DNA sequences in a cell. In a simple example, the knock-out construct is comprised of a gene, such as the CETP gene, with a deletion in a critical portion of the gene so that active protein cannot be expressed therefrom. Alternatively, a number of termination codons can be added to the native gene to cause early termination of the protein or an intron junction can be inactivated. In a typical knock-out construct, some portion of the gene is replaced with a selectable marker (such as the neo gene) so that the gene can be represented as follows: CETP 5'/neo/ CETP 3', where 5' and 3', refer to genomic or cDNA sequences which are, respectively, upstream and downstream relative to a portion of the CETP gene and where neo refers to a neomycin resistance gene. In another knock-out construct, a second selectable marker is added in a flanking position so that the gene can be represented as: CETP 5'/neo/CETP 3'/TK, where TK is a thymidine kinase gene which can be added to either the 5' or 3' sequence of the preceding construct and which further can be selected against (i.e., is a negative selectable marker) in appropriate media. This two-marker construct allows the selection of homologous recombination events, which removes the flanking TK marker, from non-homologous recombination events which typically retain the TK sequences. The gene deletion and/or replacement can be from the exons, introns, especially intron junctions, and/or the regulatory regions such as promoters;
"linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern;
"marker" refers to a sequence in the genome that is known to vary among individuals;
"modulate" refers to the ability of a substance to affect bioactivity. When applied to a CETP bioactivity, an agonist or antagonist can modulate bioactivity for example by agonizing or antagonizing a CETP synthesis, or cholesteryl ester transferase activity;
"non-human animal" of the invention includes mammals such as rodents, non-human primates, sheep, dogs, cows, goats, etc., amphibians, such as members of the Xenopus genus, and transgenic avians (e.g., chickens, birds, etc.). The term "chimeric animal" is used herein to refer to animals in which the recombinant gene is found, or in which the recombinant gene is expressed in some but not all cells of the animal. The term "tissue-specific chimeric animal" indicates that one of the recombinant CETP genes is present and/or expressed or disrupted in some tissues but not others. The term "non-human mammal" refers to any member of the class Mammalia, except for humans;
"nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g., peptide nucleic acids) and as applicable to the embodiment being described, single (sense or antisense) and double-stranded polynucleotides;
"peripheral vascular disease" ("PVD") is a cardiovascular disease resulting from the blockage of the peripheral (i.e., non-coronary) arteries. Blockage can occur suddenly, by mechanisms such as plaque rupture or embolization, as occurs in fragile plaque disease. Blockage can occur progressively, with narrowing of the artery via myointimal hyperplasia and plaque formation, as in occlusive disease. Blockage can be complete or partial. Those clinical signs and symptoms resulting from the blockage of peripheral arteries are manifestations of peripheral vascular disease. Manifestations of peripheral vascular diseases include, inter alia, claudication, ischemia, intestinal angina, vascular-based renal insufficiency, transient ischemic attacks, aneurysm formation, peripheral embolization and stroke. Ischemic cerebrovascular disease is a type of peripheral vascular disease;
"polymorphism" refers to the coexistence of more than one form of a gene, or portion (e.g., allelic variant) thereof, in a given population. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long;
"propensity to disease," also "predisposition" or "susceptibility" to disease or any similar phrase, means that certain alleles are hereby discovered to be associated with or predictive of a subject's incidence of developing a particular disease (herein, a cardiovascular disease). The alleles are thus over-represented in frequency in individuals with disease as compared to healthy individuals. Thus, these alleles can be used to predict disease even in pre-symptomatic or pre-diseased individuals. These alleles are understood to relate to the disorder underlying the disease;
"risk factor" is a factor identified to be associated with an increased risk. A risk factor for a cardiovascular disorder or a cardiovascular disease is any factor identified to be associated with an increased risk of developing those conditions or of worsening those conditions. A risk factor can also be associated with an increased risk of an adverse clinical event or an adverse clinical outcome in a patient with a cardiovascular disorder. Risk factors for cardiovascular disease include smoking, adverse lipid profiles, elevated lipids or cholesterol, diabetes, hypertension, hypercoagulable states, elevated homocysteine levels, and lack of exercise. Carrying a particular polymorphic allele is a risk factor for a particular cardiovascular disorder, and is associated with an increased risk of the particular disorder;
"small molecule" as used herein, is meant to refer to a composition, which has a molecular weight of less than about 5kD and most preferably less than about 4kD. Small molecules can be nucleic acids, peptides, peptidomimetics, carbohydrates, lipids or other organic or inorganic molecules;
"specifically hybridizes" or "specifically detects" refers to the ability of a nucleic acid molecule to hybridize to at least approximately 6 consecutive nucleotides of a sample nucleic acid;
"stenosis," as understood herein refers to a narrowing of an artery as seen in occlusive disorder or in restenosis. Stenosis can be accompanied by those symptoms reflecting a decrease in blood flow past the narrowed arterial segment, in which case the disorder giving rise to the stenosis is termed a disease (i.e., occlusive disease or restenosis disease). Stenosis can exist asymptomatically in a vessel, to be detected only by a diagnostic intervention such as an angiography or a vascular lab study;
"transcriptional regulatory sequence" is a generic term used throughout the specification to refer to DNA sequences, such as initiation signals, enhancers, and promoters, which induce or control transcription of protein coding sequences with which they are operably linked;
"transgene" means a nucleic acid sequence (encoding, e.g., one of the CETP polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid;
"transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of a CETP polypeptide, e.g., either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination or antisense techniques. The term is intended to include all progeny generations. Thus, the founder animal and all F1, F2, F3, and so on, progeny thereof are included;
"treating" as used herein is intended to encompass curing as well as ameliorating at least one symptom of a disease or at least one abnormality associated with a disorder. Treating a cardiovascular disorder can take place by administering a cardiovascular disorder therapeutic. Treating a cardiovascular disorder can also take place by modifying risk factors that are related to the cardiovascular disorder;
"treatment plan" refers to at least one intervention undertaken to modify the effect of a risk factor upon a patient. A treatment plan for a cardiovascular disorder or disease can address those risk factors that pertain to cardiovascular disorders or diseases. A treatment plan can include an intervention that focuses on changing patient behavior, such as stopping smoking. A treatment plan can include an intervention whereby a therapeutic agent is administered to a patient. As examples, cholesterol levels can be lowered with proper medication, and diabetes can be controlled with insulin. Nicotine addiction can be treated by withdrawal medications. A treatment plan can include an intervention that is diagnostic. The presence of the risk factor of hypertension, for example, can give rise to a diagnostic intervention whereby the etiology of the hypertension is determined. After the reason for the hypertension is identified, further treatments may be administered;
"vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto; and
"wild-type allele" or "normal allele" refer to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes. In general a "wild-type allele" is the most common allele in a population.

The present invention is based, at least in part, on the identification of novel alleles of the CETP gene, which alleles are useful for identifying patients with a change in the levels of CETP protein and/or liproproteins such as HDL, LDL, IDL and VLDL. Therefore, detection of these alleles in a subject indicates that the subject has an altered susceptibility to development (increased or decreased) of a cardiovascular disorder. Further, detection of the alleles aids in determining the choice of therapeutic which will be optimally useful for patients with a particular CETP haplotype.

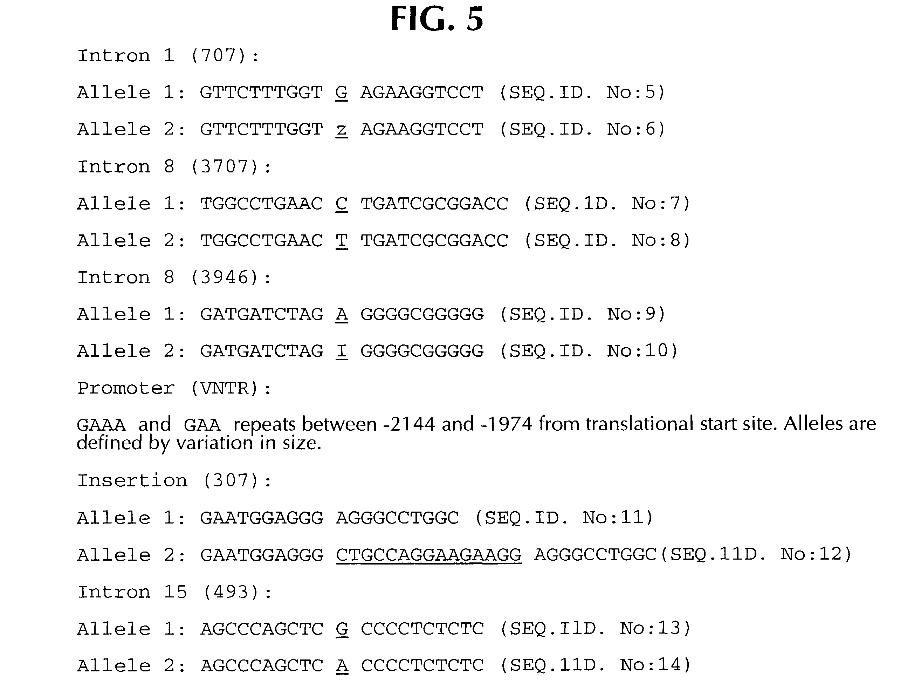

A first embodiment of the invention relates to CETP polymorphic loci and alleles including: intron 1 (707); intron 8 (3707); intron 8 (3946); promoter (VNTR); insertion (307); and intron 15 (493) (see Figures 5 and 6).

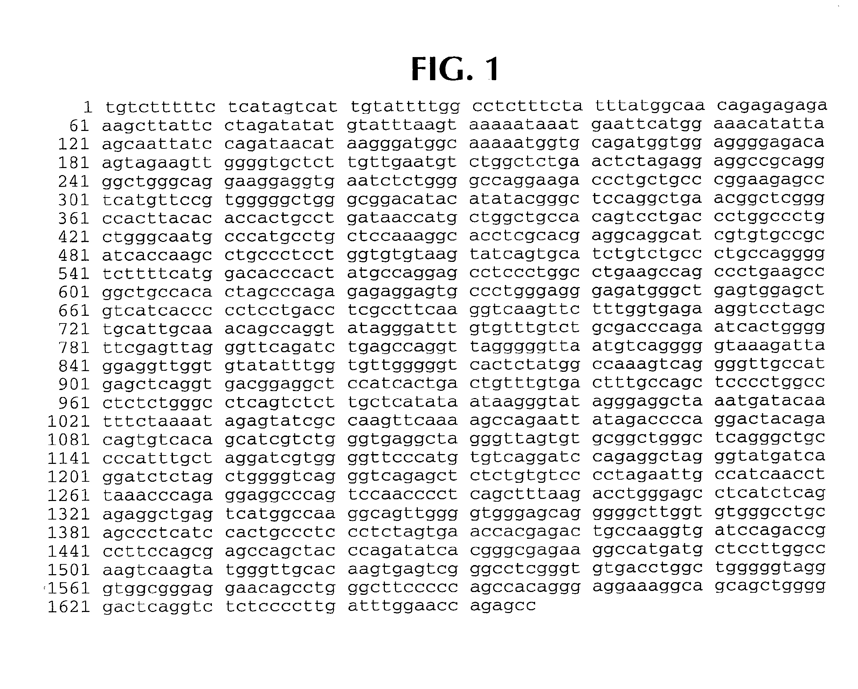

The intron 1 (707) polymorphism is a G to A mutation at nucleotide 707 of intron 1 of the CETP gene. The number designation is relative to the numbering based on GenBank sequence Accession number M32992 (Figure 1). The polymorphism is flanked by sequences GTTCTTTGGT G/A AGAAGGTCCT. It can be detected by an Hph I RFLP and is in complete linkage disequilibrium with the Taq IB polymorphism. The intron 1 (707) polymorphism was discovered by sequence analysis of intron 1 of the CETP gene.

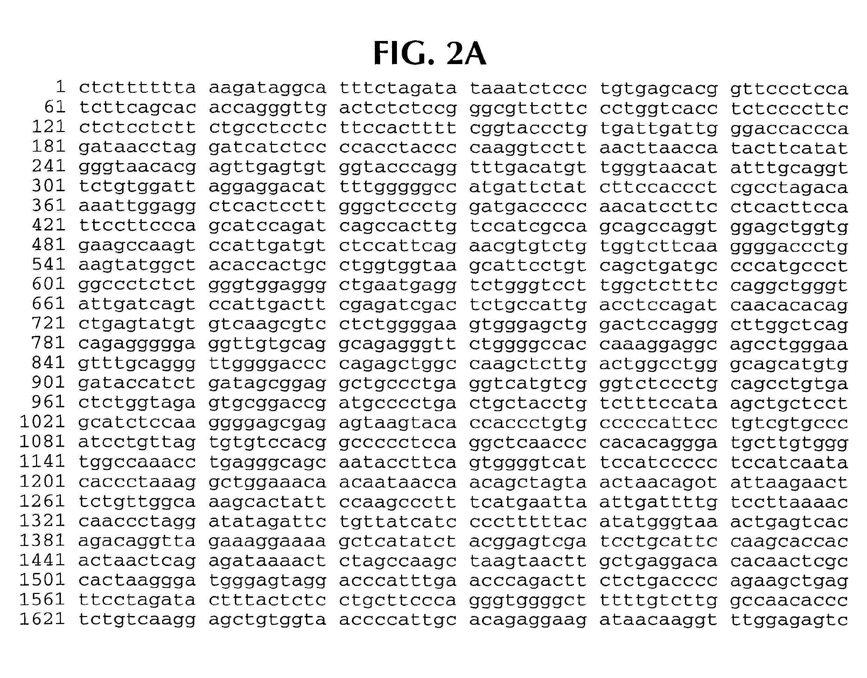

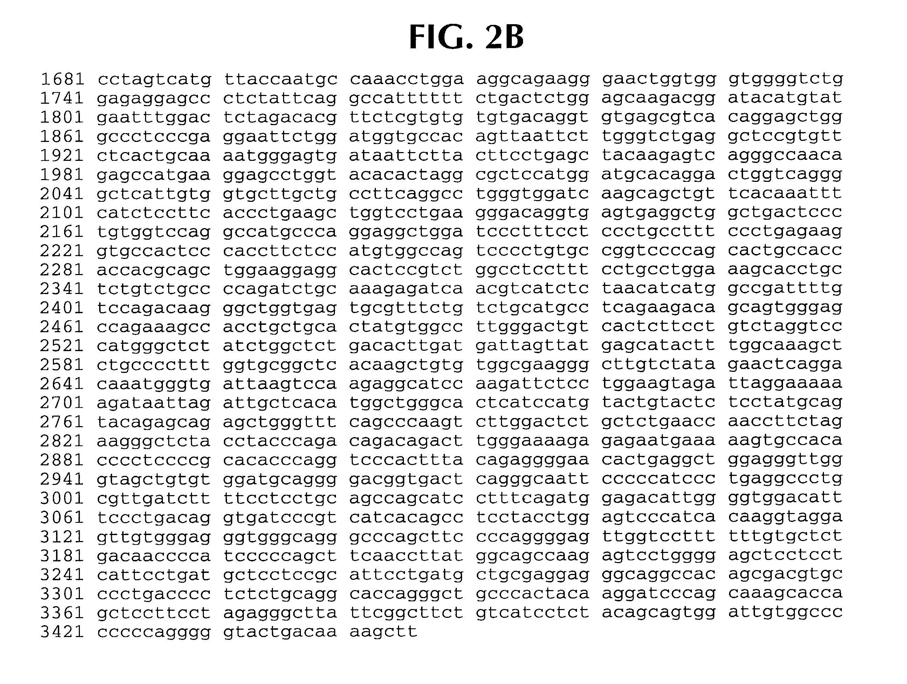

The intron 8 (3707) polymorphism is a C to T mutation at nucleotide 3707 of intron 8 of the CETP gene. The polymorphism lies 261 bp downstream from the last nucleotide (3446) covered by GenBank sequence M32993 (Figure 2). It is flanked by sequences TGGCCTGAAC C/T TGATCGCAGGACC. The intron 8 (3707) polymorphism was discovered by sequence analysis of previously unreported areas of intron 8 of the CETP gene.

The intron 8 (3946) polymorphism is an A to T mutation at nucleotide 3946 of intron 8 of the CETP gene. The polymorphism lies 500 bp downstream from the last nucleotide covered by GenBank sequence M32993 (Figure 2). It is flanked by sequences GATGATCTAG A/T GGGGCGGGGG. The intron 8 (3946) polymorphism was also discovered by sequence analysis of previously unreported areas of intron 8 of the CETP gene.

The promoter (VNTR) polymorphism is characterized by variable number of GAAA repeats clustered within a GA-rich region of the CETP promoter. Differences in PCR fragment sizes were observed among several individuals. Sequence analysis has confirmed varying number of repeats in the area of the promoter between -2144 and -1974 from the transcriptional start site of CETP. Genotype data is suggestive that increased CETP mass is observed in individuals who also possess increased number of repeats.

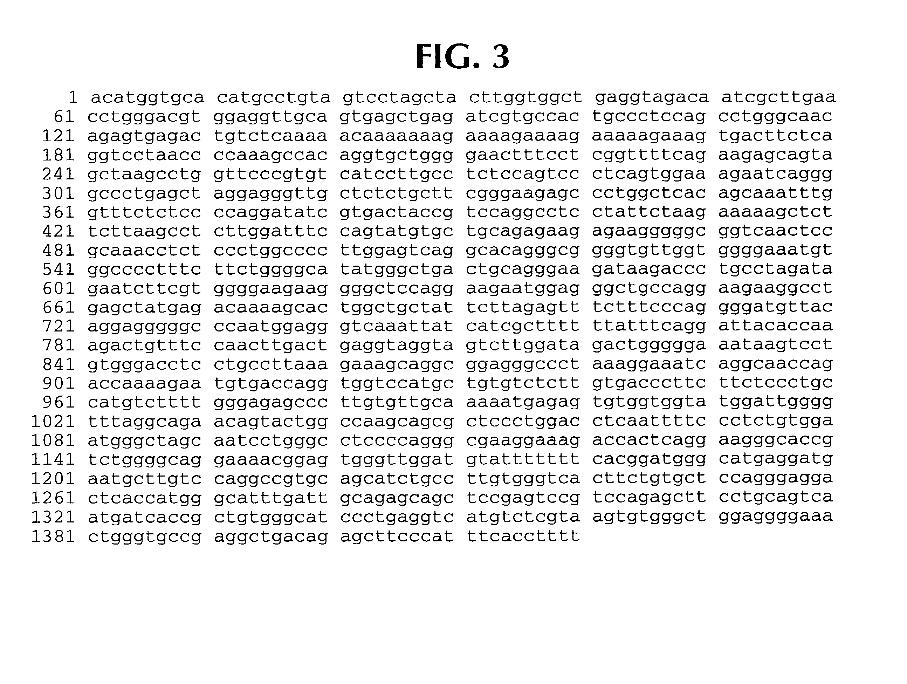

The insertion (307) polymorphism is characterized by a 15 bp insertion in intron 12 of the CETP gene. The insertion occurs at nucleotides 643-657 based on the numbering provided by GenBank sequence Accession number M32997 (Figure 3). It is flanked by sequences GAATGGAGGG -CTGCCAGGAAGAAGG - AGGGCCTGGC. The polymorphism is flanked to the right by a 99 bp sequence erroneously omitted from the GenBank sequence of intron 12. The insertion (307) variant was also identified to be in complete linkage disequilibrium with a 3' UTR polymorphism, SNP 565, which is highly correlated to HDL levels (See Examples).

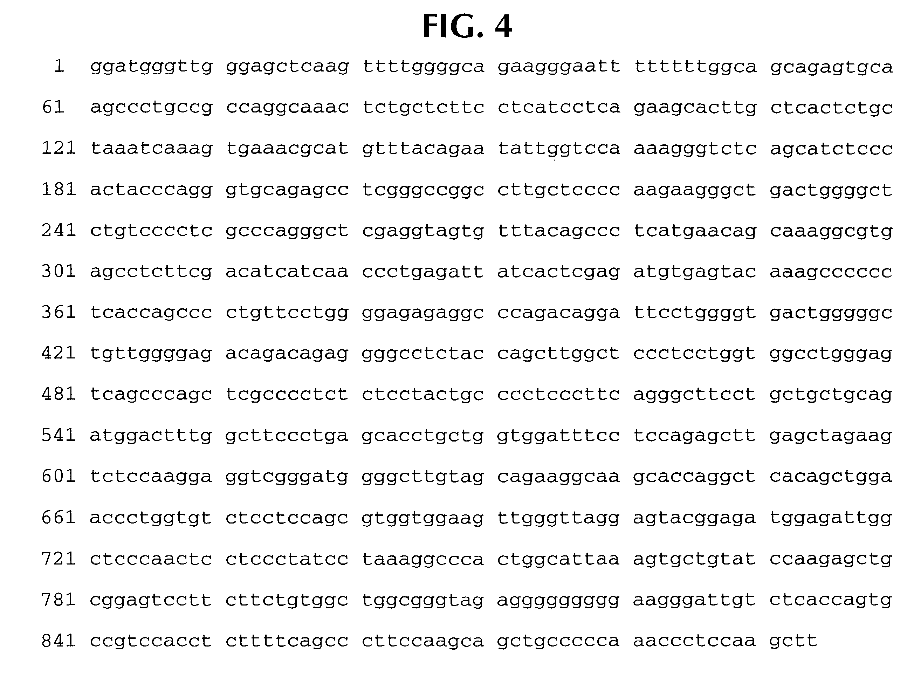

The intron 15 (493) polymorphism is a G to A mutation at nucleotide 493 of intron 15 of the CETP gene. The number designation is relative to the numbering based on GenBank sequence Accession number M32998 (Figure 4). It is flanked by sequences AGCCCAGCTC G/A CCCCTCTCTC. The intron 15 (493) polymorphism was identified by sequence analysis of intron 15.

In addition to the allelic patterns described above, as described herein, one of skill in the art can readily identify other alleles (including polymorphisms and mutations) that are in linkage disequilibrium with an allele associated with a cardiovascular disorder. For example, a nucleic acid sample from a first group of subjects without a cardiovascular disorder can be collected, as well as DNA from a second group of subjects with a cardiovascular disorder. The nucleic acid sample can then be compared to identify those alleles that are over-represented in the second group as compared with the first group, wherein such alleles are presumably associated with a cardiovascular disorder. Alternatively, alleles that are in linkage disequilibrium with a cardiovascular disorder associated allele can be identified, for example, by genotyping a large population and performing statistical analysis to determine which alleles appear more commonly together than expected. Preferably the group is chosen to be comprised of genetically related individuals. Genetically related individuals include individuals from the same race, the same ethnic group, or even the same family. As the degree of genetic relatedness between a control group and a test group increases, so does the predictive value of polymorphic alleles which are ever more distantly linked to a disease-causing allele. This is because less evolutionary time has passed to allow polymorphisms which are linked along a chromosome in a founder population to redistribute through genetic cross-over events. Thus, race-specific, ethnic-specific, and even family-specific diagnostic genotyping assays can be developed to allow for the detection of disease alleles which arose at ever more recent times in human evolution, e.g., after divergence of the major human races, after the separation of human populations into distinct ethnic groups, and even within the recent history of a particular family line.

Linkage disequilibrium between two polymorphic markers or between one polymorphic marker and a disease-causing mutation is a meta-stable state. Absent selective pressure or the sporadic linked reoccurrence of the underlying mutational events, the polymorphisms will eventually become disassociated by chromosomal recombination events and will thereby reach linkage equilibrium through the course of human evolution. Thus, the likelihood of finding a polymorphic allele in linkage disequilibrium with a disease or condition may increase with changes in at least two factors: decreasing physical distance between the polymorphic marker and the disease-causing mutation, and decreasing number of meiotic generations available for the dissociation of the linked pair. Consideration of the latter factor suggests that, the more closely related two individuals are, the more likely they will share a common parental chromosome or chromosomal region containing the linked polymorphisms and the less likely that this linked pair will have become unlinked through meiotic cross-over events occurring with each generation. As a result, the more closely related two individuals are, the more likely it is that widely spaced polymorphisms may be co-inherited. Thus, for individuals related by common race, ethnicity or family, the reliability of ever more distantly spaced polymorphic loci can be relied upon as an indicator of inheritance of a linked disease-causing mutation.

The oligonucleotides present in one embodiment of a kit according to the present invention may be used for amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization).

Appropriate probes may be designed to hybridize to specific regions of the CETP gene. Primers useful for amplifying regions of the CETP gene will be apparent to one of skill in the art in light of the present disclosure. Reasonable primers include those which hybridize within about 1 kb of the designated primer, and which further are anywhere from about 17 bp to about 27 bp in length. A general guideline for designing primers for amplification of unique human chromosomal genomic sequences is that they possess a melting temperature of at least about 50°C, wherein an approximate melting temperature can be estimated using the formula Tₘₑₗₜ = [2 x (# of A or T) + 4 x (# of G or C)]. These probes may incorporate other regions of the relevant genomic locus, including intergenic sequences. Indeed, the CETP gene spans some 25,000 base pairs and, assuming an average of one single nucleotide polymorphism every 1,000 base pairs, includes some 25 SNP loci alone. Yet other polymorphisms available for use with the immediate invention are obtainable from various public sources. For example, the human genome database collects intragenic SNPs, is searchable by sequence and currently contains approximately 2,700 entries (http://hgbase.interactiva.de). Also available is a human polymorphism database maintained by the Massachusetts Institute of Technology (MIT SNP database http://www.genome.wi.mit.edu/SNP/human/index.html)). From such sources, SNPs as well as other human polymorphisms may be found.

Accordingly, the nucleotide segments of the invention may be used for their ability to selectively form duplex molecules with complementary stretches of the CETP gene region. The design of appropriate probes for this purpose requires consideration of a number of factors. For example, fragments having a length of between 10, 15, or 18 nucleotides to about 20, or to about 30 nucleotides, will find particular utility. Longer sequences, e.g., 40, 50, 80, 90, 100, even up to full length, are even more preferred for certain embodiments. Lengths of oligonucleotides of at least about 18 to 20 nucleotides are well accepted by those of skill in the art as sufficient to allow sufficiently specific hybridization so as to be useful as a molecular probe. Furthermore, depending on the application envisioned, one will desire to employ varying conditions of hybridization to achieve varying degrees of selectivity of probe towards target sequence. For applications requiring high selectivity, one will typically desire to employ relatively stringent conditions to form the hybrids. For example, relatively low salt and/or high temperature conditions, such as provided by 0.02 M-0.15M NaCI at temperatures of about 50°C to about 70°C. Such selective conditions may tolerate little, if any, mismatch between the probe and the template or target strand.

Other alleles or other indicia of a vascular disorder can be detected or monitored in a subject in conjunction with detection of the alleles described above, for example, identifying vessel wall thickness (e.g., as measured by ultrasound), or whether the subject smokes, drinks, is overweight, is under stress, has elevated cholesterol or low cholesterol has elevated Lp(a), or exercises.

Another embodiment of the invention relates to linkage disequilibrium analysis and haplotype patterns, including pattern 1 (also called the 11121 pattern) and pattern 2 (also called the 22212 pattern) (Table 1; see Examples).

**Table 1:**

| CETP Haplotypes | | | | | |
|---|---|---|---|---|---|
| **Haplotype** | **Locus and Allele** | | | | |
| | intron 1 (TaqIB) | intron 8 (Mspl) | intron 12 (insertion 307) | exon 14 (Rsal) | exon 16 (SNP565) |
| Pattern 1 | 1 | 1 | 1 | 2 | 1 |
| Pattern 2 | 2 | 2 | 2 | 1 | 2 |

Haplotype pattern 1 is associated with higher CETP levels and lower HDL levels, while the haplotype pattern 2 is associated with lower CETP levels and higher HDL levels. Therefore, Haplotype 1 is indicative of risk factors commonly associated with the development of cardiovascular disorders.

Still another embodiment of the invention relates to correlation of a CETP haplotype pattern with susceptibility to disease, partiuclarly cardiovascular disorders. Polymorphisms in the CETP gene locus are associated with alterations in the levels of CETP protein and HDL levels, which have been shown to be risk factors for cardiovascular disorders, particularly coronary artery disease. CETP polymorphisms may result in increased or decreased susceptibility to cardiovascular disorders.

Another embodiment of the invention relates to correlation of a particular CETP haplotype with identification of an optimal choice of therapeutic for a patient, particularly a patient suffering from a cardiovascular disorder.

Many methods are available for detecting specific alleles at human polymorphic loci. The preferred method for detecting a specific polymorphic allele will depend, in part, upon the molecular nature of the polymorphism. For example, the various allelic forms of the polymorphic locus may differ by a single base-pair of the DNA. Such single nucleotide polymorphisms (or SNPs) are major contributors to genetic variation, comprising some 80% of all known polymorphisms, and their density in the human genome is estimated to be on average 1 per 1,000 base pairs. SNPs are most frequently biallelic- occurring in only two different forms (although up to four different forms of an SNP, corresponding to the four different nucleotide bases occurring in DNA, are theoretically possible). Nevertheless, SNPs are mutationally more stable than other polymorphisms, making them suitable for association studies in which linkage disequilibrium between markers and an unknown variant is used to map disease-causing mutations. In addition, because SNPs typically have only two alleles, they can be genotyped by a simple plus/minus assay rather than a length measurement, making them more amenable to automation.

A variety of methods are available for detecting the presence of a particular single nucleotide polymorphic allele in an individual. Advancements in this field have provided accurate, easy, and inexpensive large-scale SNP genotyping. Most recently, for example, several new techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips (Santa Clara, CA). These methods require amplification of the target genetic region, typically by PCR. Still other newly developed methods, based on the generation of small signal molecules by invasive cleavage followed by mass spectrometry or immobilized padlock probes and rolling-circle amplification, might eventually eliminate the need for PCR. Several of the methods known in the art for detecting specific single nucleotide polymorphisms are summarized below. The method of the present invention is understood to include all available methods.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO 91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., *Nucl. Acids. Res.* 17:7779-7784 (1989); Sokolov, B. P., *Nucl. Acids Res.* 18:3671 (1990); Syvanen, A. -C., et al., *Genomics* 8:684-692 (1990); Kuppuswamy, M. N. et al., *Proc. Natl. Acad. Sci. (U.S.A.)* 88:1143-1147 (1991); Prezant, T. R. et al., *Hum. Mutat.* 1:159-164 (1992); Ugozzoli, L. et al., *GATA* 9:107-112 (1992); Nyren, P. et al., *Anal. Biochem.* 208:171-175 (1993)). These methods differ from GBA™ in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., *Amer. J. Hum. Genet.* 52:46-59 (1993)).

For mutations that produce premature termination of protein translation, the protein truncation test (PTT) offers an efficient diagnostic approach (Roest, et. al., (1993) *Hum. Mol. Genet.* 2:1719-21; van der Luijt, et. al., (1994) *Genomics* 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential in vitro transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment, the DNA sample is obtained from a bodily fluid, e.g., blood, obtained by known techniques (e.g., venipuncture) or saliva. Alternatively, nucleic acid tests can be performed on dry samples (e.g., hair or skin). When using RNA or protein, the cells or tissues that may be utilized must express the CETP protein.

Diagnostic procedures may also be performed *in situ* directly upon tissue sections (fixed and/or frozen) of patient tissue obtained from biopsies or resections, such that no nucleic acid purification is necessary. Nucleic acid reagents may be used as probes and/or primers for such *in situ* procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

A preferred detection method is allele specific hybridization using probes overlapping a region of at least one allele of a CETP haplotype and having about 5, 10, 20, 25, or 30 nucleotides around the mutation or polymorphic region. In a preferred embodiment of the invention, several probes capable of hybridizing specifically to other allelic variants involved in a cardiovascular disorder are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. *Human Mutation* 7:244 (1996). In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, PCR, polymerase chain reaction of specific alleles (ASA), ligase chain reaction (LCR), nested polymerase chain reaction, self sustained sequence replication (Guatelli, J.C. et al., *Proc. Natl. Acad. Sci. (USA)* 87:1874-1878 (1990)), transcriptional amplification system (Kwoh, D.Y. et al., *Proc. Natl. Acad. Sci. (USA)* 86:1173-1177 (1989)), and Q- Beta Replicase (Lizardi, P.M. et al., *Bio*/*Technology* 6:1197 (1988)).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of a CETP haplotype under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of a CETP haplotype is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety of sequencing reactions known in the art can be used to directly sequence the allele. Exemplary sequencing reactions include those based on techniques developed by Maxim and Gilbert *(Proc. Natl Acad Sci (USA)* 74:560 (1977)) or Sanger (Sanger et al., *Proc. Nat. Acad. Sci (USA)* 74:5463 (1977)). It is also contemplated that any of a variety of automated sequencing procedures may be utilized when performing the subject assays (see, for example *Biotechniques* 19:448 (1995)), including sequencing by mass spectrometry (see, for example PCT publication WO 94/16101; Cohen et al., *Adv. Chromatogr.* 36:127-162 (1996); and Griffin et al., *Appl. Biochem. Biotechnol.* 38:147-159 (1993)). It will be evident to one of skill in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al., *Science* 230:1242 (1985)). In general, the art technique of "mismatch cleavage" starts by providing heteroduplexes formed by hybridizing (labeled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al., *Proc. Natl. Acad. Sci. (USA)* 85:4397 (1988); and Saleeba et al., *Methods Enzymol.* 217:286-295 (1992). In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of E. coli cleaves A at G/A mismatches and the thymidine DNA glycosylase from HeLa cells cleaves T at G/T mismatches (Hsu et al., *Carcinogenesis* 15:1657-1662 (1994)). According to an exemplary embodiment, a probe based on an allele of a CETP haplotype is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify a CETP allele. For example, single strand conformation polymorphism (SSCP) may be used to detect differences in electrophoretic mobility between mutant and wild type nucleic acids (Orita et al., *Proc Natl. Acad. Sci (USA)* 86:2766 (1989), see also Cotton, *Mutat. Res*. 285:125-144 (1993); and Hayashi, *Genet. Anal. Tech. Appl.* 9:73-79 (1992)). Single-stranded DNA fragments of sample and control CETP alleles are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al., *Trends Genetics* 7:5 (1991)).

In yet another embodiment, the movement of alleles in polyacrylamide gels containing a gradient of denaturant is assayed using denaturing gradient gel electrophoresis (DGGE) (Myers et al., *Nature* 313:495 (1985)). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner, *Biophys. Chem.* 265:12753 (1987)).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al., *Nature* 324:163 (1986)); Saiki et al., *Proc. Natl. Acad. Sci. (USA)* 86:6230 (1989)). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al., *Nucleic Acids Res.* 17:2437-2448 (1989)) or at the extreme 3' end of one primer where, under appropriate conditions, mismatch can prevent, or reduce polymerase extension (Prossner, *Tibtech* 11:238 (1993). In addition it may be desirable to introduce a novel restriction site in the region of the mutation to create cleavage-based detection (Gasparini et al., *Mol. Cell* Probes 6:1 (1992)). It is anticipated that in certain embodiments amplification may also be performed using Taq ligase for amplification (Barany, *Proc. Natl. Acad. Sci. (USA)* 88:189 (1991)). In such cases, ligation will occur only if there is a perfect match at the 3' end of the 5' sequence making it possible to detect the presence of a known mutation at a specific site by looking for the presence or absence of amplification.

In still another embodiment, an allelic variant can also be identified by alterations in restriction enzyme cleavage patterns through restriction fragment length polymorphism (RFLP) analysis. For example, sample and control DNA is isolated, amplified (optionally), digested with one or more restriction endonucleases, and fragment length sizes are determined by size fractionation (e.g., liquid chromatography or gel electrophoresis).

In another embodiment, identification of the allelic variant is carried out using an oligonucleotide ligation assay (OLA), as described, e.g., in U.S. Pat. No. 4,998,617 and in Landegren, U. et al. (*Science* 241:1077-1080 (1988)). The OLA protocol uses two oligonucleotides which are designed to be capable of hybridizing to abutting sequences of a single strand of a target. One of the oligonucleotides is linked to a separation marker, e.g., biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et. al., *Proc. Natl. Acad. Sci. (USA)* 87:8923-27 (1990)). In this method, PCR is used to achieve the exponential amplification of target DNA, which is then detected using OLA.

Several techniques based on this OLA method have been developed and can be used to detect CETP alleles. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. (*Nucleic Acids Res.* 24: 3728 (1996)), OLA combined with PCR permits typing of two alleles in a single microtiter well. By marking each of the allele-specific primers with a unique hapten, i.e., digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Another embodiment of the invention is directed to kits for detecting a predisposition for developing a cardiovascular disorder, either due to the occlusion of an artery or due to the formation of fragile plaque, or due to the formation of restenosis. This kit may contain one or more oligonucleotides, including 5' and 3' oligonucleotides that hybridize 5' and 3' to at least one allele of a CETP haplotype. PCR amplification oligonucleotides should hybridize between 25 and 2500 base pairs apart, preferably between about 100 and about 500 bases apart, in order to produce a PCR product of convenient size for subsequent analysis.

The design of oligonucleotides for use in the amplification and detection of CETP polymorphic alleles by the method of the invention is facilitated by the availability of both updated sequence information from human chromosome 16 (16q12 - 16q21), which contains the human CETP locus, and updated human polymorphism information available for this locus. Suitable primers for the detection of a human polymorphism in these genes can be readily designed using this sequence information and standard techniques known in the art for the design and optimization of primers sequences. Optimal design of such primer sequences can be achieved, for example, by the use of commercially available primer selection programs such as Primer 2.1, Primer 3 or GeneFisher.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, JW, et al., *J. of Invest. Dermatol.* 103:387-389 (1994)) and the like; DNA purification reagents such as Nucleon™ kits, lysis buffers, proteinase solutions and the like; PCR reagents, such as 10x reaction buffers, thermostable polymerase, dNTPs, and the like; and allele detection means such as the Hinfl restriction enzyme, allele specific oligonucleotides, and degenerate oligonucleotide primers for nested PCR from dried blood.

Various methods for measuring levels of HDL in a blood sample are well known in the art, such as clotting, centrifugation, ultracentrifugation, electrophoresis, etc. Additionally, a variety of commercially available kits are available for measuring HDL levels. A specific example of an assay for measuring HDL levels is the dextran sulfate MnCl₂ precipitation method (Warnick et al., Clinical Chemistry, 28, 1385-1397(1982)). Briefly, a sample of blood plasma is mixed with an HDL-cholesterol reagent (Sigma 352-3) and reacted for 5 minutes. The reaction is then centrifuged at 3,000 x g for 5 minutes and a fraction of the supernatant is mixed with an enzyme reagent for determining cholesterol (Sigma 352-50). The mixture is reacted at 37°C for 5 minutes with stirring; an aliquot is taken and its O.D. at 500 nm is determined with a spectrometer. The concentration of HDL cholesterol is calculated by comparing the result with a standard curve.

Various methods for measuring levels of CETP in a blood sample are well known in the art, such as ELISA, RIA, etc. (see e.g., Clark et al., J. Lipid Res. 36: 876-889 (1995); Brown et al., Nature 342: 448-451 (1989); U.S Patent No. 6,140,474). Additionally, a variety of commercially available kits are available for measuring CETP levels. A specific method for measuring CETP levels (or CETP mass) in a blood serum sample is the two-site immunoassay (Mezdour et al., Clin. Chem. 40: 593-597 (1994)).

CETP activity may be measured using a variety of methods. In general the methods involve incubation of CETP with labeled cholesteryl ester in the presence of HDL and VLDL and/or LDL. The amount of CETP activity is determined by isolating the VLDL/LDL component from the reaction mixture and determining the amount of label in the low density fraction. The CE may be labeled, for example, with radioactivity or fluorescent labels, and the low density fraction may be isolated, for example, by ultra-centrifugation, precipitation, etc. (see e.g., Nichols A.V., et al., *J*. *Lipid Res.* 6: 206 (1965); Pattnaik, N.M. et al., *Bioch. Biophys. Acta* 530: 428 (1978); Dousset, N. et al., *Clin. Chem.* 38: 306 (1982); Milner, T.G. et al., *Biochim. Biophys. Acta* 1082: 71 (1991) and Brocia, R.W. et al. US Pat. No. 5,618,683).

Knowledge of the particular CETP alleles associated with a susceptibility to developing a cardiovascular disorder, alone or in conjunction with information on other genetic defects contributing to a cardiovascular disorder allows a customization of the prevention or treatment in accordance with the individual's genetic profile, the goal of "pharmacogenomics".

Accordingly, therapeutics that address the particular molecular basis of a cardiovascular disease in a subject may be developed based upon genotype analysis. Comparison of an individual's CETP profile to the population profile for a cardiovascular disorder, permits the selection or design of drugs or other therapeutic regimens that are expected to be safe and efficacious for a particular patient or patient population (i.e., a group of patients having the same genetic alteration).

In particular, determination of a CETP haplotype is important for assessing a subjects susceptibility to development of a cardiovascular disorder due to a change in the levels or activity of CETP and/or the levels of lipoproteins such as HDL, LDL, IDL and VLDL. For example, subjects having an allele of pattern 1 tend to have a lower level of HDL which may render them more at risk for developing cardiovascular disorders. Furthermore, the genotypic information allows the clinician to judge treatments that might be more effective. For example, in those patients that have a cardiovascular disorder and an allele of pattern 1, the cardiovascular disorder is caused in part by the relatively higher CETP concentration. Therefore, pharmaceuticals that are CETP antagonists are likely to have a beneficial effect. By the same token, patients with pattern 2 presenting with a cardiovascular disorder will benefit less from a CETP antagonist because the CETP levels are already low in these patients.

In addition, the ability to target populations expected to show the highest clinical benefit, based on genetic profile can: 1) enable the repositioning of drugs already marketed for prevention or treatment of cardiovascular disorder; 2) enable the rescue of drug candidates whose clinical development has been discontinued as a result of limitations which are patient subgroup-specific; and 3) facilitate development for candidate therapeutics and more optimal drug labeling (e.g., since measuring the effect of various doses of an agent on a vascular disorder causative mutation is useful for optimizing effective dose).

The treatment of an individual with a particular therapeutic can be monitored by determining protein (e.g., CETP), mRNA and/or transcriptional level. CETP activity can also be monitored. Depending on the level detected, the therapeutic regimen can then be maintained or adjusted (increased or decreased in dose). In a preferred embodiment, the effectiveness of treating a subject with an agent comprises the steps of: (i) obtaining a preadministration sample from a subject prior to administration of the agent; (ii) detecting the level or amount of CETP activity, protein or mRNA in the preadministration sample; (iii) obtaining one or more post-administration samples from the subject; (iv) detecting the level of expression or activity of the CETP activity, protein, or mRNA in the post-administration sample; (v) comparing the level of the CETP measurement in the preadministration sample with the corresponding CETP measurement in the postadministration sample, respectively; and (vi) altering the administration of the agent to the subject accordingly.

Cells of a subject may also be obtained before and after administration of a therapeutic to detect the level of expression of genes other than a CETP gene to verify that the therapeutic does not increase or decrease the expression of genes which could be deleterious. This can be done, e.g., by using the method of transcriptional profiling. Thus, mRNA from cells exposed in vivo to a therapeutic and mRNA from the same type of cells that were not exposed to the therapeutic could be reverse transcribed and hybridized to a chip containing DNA from numerous genes, to thereby compare the expression of genes in cells treated and not treated with the therapeutic.

One approach to the prevention and treatment of a cardiovascular disease is to identify (a) risk factor(s) for the particular disease and administer a therapy that ameliorates or eliminates the risk factor(s).

Factors associated with the progression of atherosclerosis include Diabetes Mellitus, high blood pressure, hypercholesterolemia, high lipoprotein-a, obesity, and smoking. Of these, the factors amenable to pharmacological intervention include: i) diabetes, ii) hypertension, and iii) dyslipidemias. Examples of lipid lowering drugs include: anion exchange resins such as cholestyramine, colestipol; HMG CoA reductase inhibitors or (statins) such as simvastatin, pracastatin, cerivastatin, fluvastatin, atorvastatin, lovastatin; fibrates such as fenofibrate, bezafibrate, gemfibrozil, clofibrate, ciprofibrate; nicotinic acid and analogues: acipimox, nicofuranose; probucol which increases non-receptor mediated LDL clearance and decreases LDL oxidation; fish oils such as maxepa, omacor; and cholesterol absorption inhibitors such as pamaqueside, tiqueside, etc.

Another approach to the prevention and treatment of a cardiovascular disease is to is to identify the underlying disorder for the particular disease and administer a therapy that interferes with the progression of the underlying disorder and/or ameliorates or eliminates the symptoms and signs of the disorder. For example, a CETP polymorphism could lead to an overactive CETP protein which could result in low levels of HDL, a risk factor often associated with the development of cardiovascular disorders. Preferred treatments for such an individual would involve therapies capable of modulating CETP levels or activity.

Modulators of CETP activity can comprise any type of compound, including a protein, peptide, peptidomimetic, small molecule, or nucleic acid. Preferred agonists include nucleic acids (e.g., encoding a CETP protein or a gene that is up- or down-regulated by a CETP protein), proteins (e.g., CETP proteins or a protein that is up- or down-regulated thereby) or a small molecule (e.g., that regulates expression, binding or activity of a CETP protein). Preferred antagonists, which can be identified, for example, using the assays described herein, include nucleic acids (e.g., single (antisense) or double stranded (triplex) DNA or PNA and ribozymes), protein (e.g., antibodies) and small molecules that act to suppress or inhibit CETP transcription and/or protein activity.

Gene therapy techniques may also be used to modulate CETP activity. For example, this could include over-expression of CETP in certain tissues, or antisense treatment to decrease CETP production, or allele replacement, wherein a desirable allele of CETP is introduced to replace the defective allele.

Preferred approaches for the prevention and treatment of cardiovascular disorders involve a combined approach wherein a subject is treated with therapeutics that treat (a) risk factor(s) in conjunction with therapeutics that treat the underlying disorder. For example, combined approaches may involve treatment of the patient with at least two of the following: (i) a CETP agonist, (ii) a CETP antagonist, (iii) an HMG CoA reductase inhibitor, (iv) a change in the subjects' exercise level, diet, alcohol intake, smoking and/or dietary fat intake. A preferred combined approach is treatment of a subject with a CETP inhibitor (to increase HDL levels) in conjunction with an HMG CoA inhibitor (to decrease LDL levels).

The practice of the present invention will employ, unless otherwise indicated, conventional techniques of cell biology, cell culture, molecular biology, transgenic biology, microbiology, recombinant DNA, and immunology, which are within the skill of the art. Such techniques are described in the literature. See, for example, *Molecular Cloning A Laboratory Manual*, 2nd Ed., ed. by Sambrook, Fritsch and Maniatis (Cold Spring Harbor Laboratory Press: 1989); *DNA Cloning,* Volumes I and II (D. N. Glover ed., 1985); *Oligonucleotide Synthesis* (M. J. Gait ed., 1984); Mullis et al. U.S. Patent No: 4,683,195; *Nucleic Acid Hybridization* (*B.* D. Hames & S. J. Higgins eds. 1984); *Transcription And Translation* (B. D. Hames & S. J. Higgins eds. 1984); *Culture Of Animal Cells* (R. I. Freshney, Alan R. Liss, Inc., 1987); *Immobilized Cells And Enzymes* (IRL Press, 1986); B. Perbal, *A Practical Guide To Molecular Cloning* (1984); the treatise, *Methods In Enzymology* (Academic Press, Inc., N.Y.); *Gene Transfer Vectors For Mammalian Cells* (J. H. Miller and M. P. Calos eds., 1987, Cold Spring Harbor Laboratory); *Methods In Enzymology,* Vols. 154 and 155 (Wu et al. eds.), *Immunochemical Methods In Cell And Molecular Biology* (Mayer and Walker, eds., Academic Press, London, 1987); *Handbook Of Experimental Immunology,* Volumes I-IV (D. M. Weir and C. C. Blackwell, eds., 1986); *Manipulating the Mouse Embryo,* (Cold Spring Harbor Laboratory Press, Cold Spring Harbor, N.Y., 1986).

The published patent and scientific literature referred to herein establishes knowledge that is available to those with skill in the art. The U.S. patents, allowed applications, published foreign patent applications, and references, including GenBank database sequences, that are cited herein are hereby incorporated in their entireties by reference to the same extent as if each was specifically and individually indicated to be incorporated by reference. Any inconsistency between these publications and the present disclosure shall be resolved in favor of the present disclosure.

The following example is intended to further illustrate certain preferred embodiments of the invention and are not limiting in nature. Those skilled in the art will recognize, or be able to ascertain, using no more than routine experimentation, numerous equivalents to the specific substances and procedures described herein. Such equivalents are considered to be within the scope of this invention, and are covered by the following claims.

### EXAMPLE

### Genetic Analysis of CETP Alleles

A genetic analysis was conducted to examine possible relationships between the novel polymorphisms in the CETP gene (intron 1 (707); intron 8 (3707); intron 8 (3946); promoter (VNTR); insertion (307); and intron 15 (493)) and baseline CETP concentration and HDL levels. A linkage disequilibrium analysis was conducted to analyze the association between the novel polymorphism and certain previously identified SNP polymorphism in the CETP gene. The Taq 1, Msp 1 and Rsa 1 polymorphisms (Figure 6) have been demonstrated in the literature to be associated with modifications in CETP and HDL levels. Specifically, a B2 allele at Taq I, an M2 allele at Msp I and an R1 allele at Rsa I, have been associated with higher HDL levels and lower CETP mass. Genetic and phenotypic data were analyzed from samples of 46 patients.

### Linkage Disequilibrium

There was complete disequilibrium observed between SNP 565 and Insertion 307 for the subjects in this study, i.e., subjects had the insertion at 307 if and only if they also had one 'C' allele at SNP 565 (the V allele is wild-type). Thus, all associations found using SNP 565 are equivalent to those for Insertion 307.

Significant linkage disequilibrium was observed among several markers in the CETP gene (Table 2).

**Table 2:**

| Linkage Analysis of CETP Polymorphisms | | | |
|---|---|---|---|
| **markers** | **Test statistic for LD (Chi-square)** | **df** | **p-value** |
| Taq1 SNP 565 | 5.46 | 1 | 0.02 |
| Msp1 SNP 565 | 3.89 | 1 | 0.05 |
| Rsa1 SNP 565 | 14.3 | 1 | 0.0002 |
| Taq1 MSP1 Rsa1 SNP 565 | 36.52 | 11 | 0.0001 |

For the 4-marker haplotype involving Taq I, Msp I, Rsa I and SNP 565, a homozygous wild-type (G/G) at SNP 565 (or equivalently no insertion at 307) was observed to be in strong disequilibrium with a 112 haplotype at Taq 1, Msp I and Rsa I (i.e., wild-type at each polymorphic site). Also, the presence of a 'C' allele at SNP 565 (or an insertion at 307) was strongly associated with a 221 haplotype at Taq I, Msp I and Rsa I (i.e., mutant at each polymorphic site).

### Genotype/Phenotype Correlations

An analysis of variance (ANOVA) was carried out with each of the above listed phenotypes and markers from CETP. In addition, the Taq I/Msp I/Rsa I/SNP 565 haplotype was analyzed with CETP concentration and HDL levels. An analysis using ranks of the phenotype variables produced similar results to those presented below (Table 3).

**Table 3:**

| Statistical Analysis of Genotype/Phenotype Correlations | | |
|---|---|---|
| **Markers / haplotype** | **Phenotype** | **p-value (not corrected for multiple tests)** |
| Taq 1 | CETP concentration | 0.02 |
| Msp 1 | CETP concentration | 0.05 |
| SNP 565 | HDL | 0.0006 |
| Taq 1 Msp 1 Rsa 1 SNP 565 (number of 1121 haplotypes) | CETP concentration | 0.05 |
| Taq 1 Msp 1 Rsa 1 SNP 565 (number of 2212 haplotypes) | HDL | 0.009 |

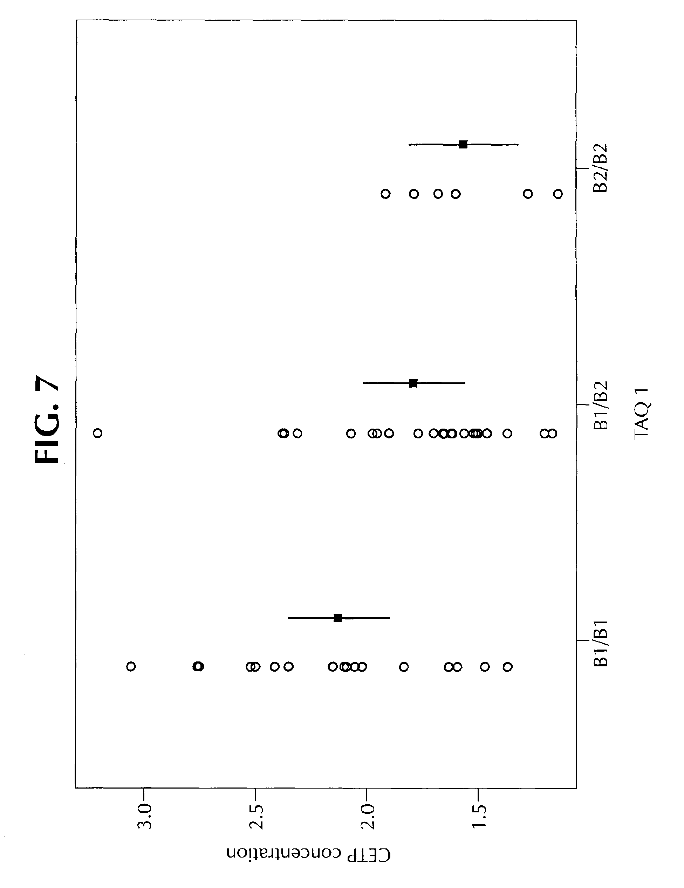

As shown in FIGURE 7, the presence of an allele containing the mutation at the Taq 1 polymorphic site in the CETP gene (the B2 allele) correlated with decreased CETP concentrations in the subjects (B1/B1 > B1/B2 > B2/B2).

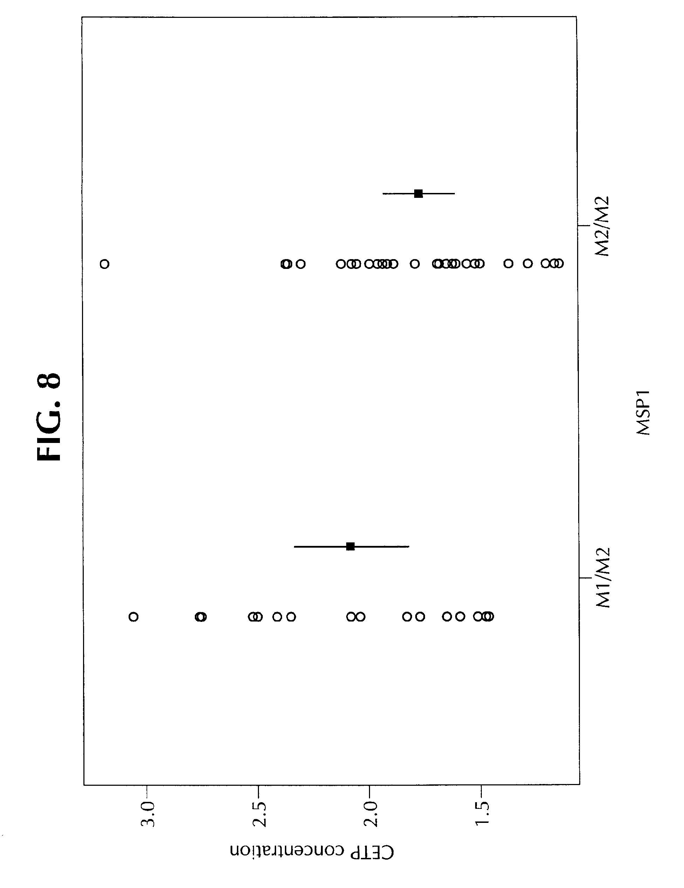

As shown in FIGURE 8, the presence of an allele containing the mutation at the Msp 1 polymorphic site in the CETP gene (the M2 allele) correlated with decreased CETP concentrations in the subjects (M1/M2 > M2/M2).

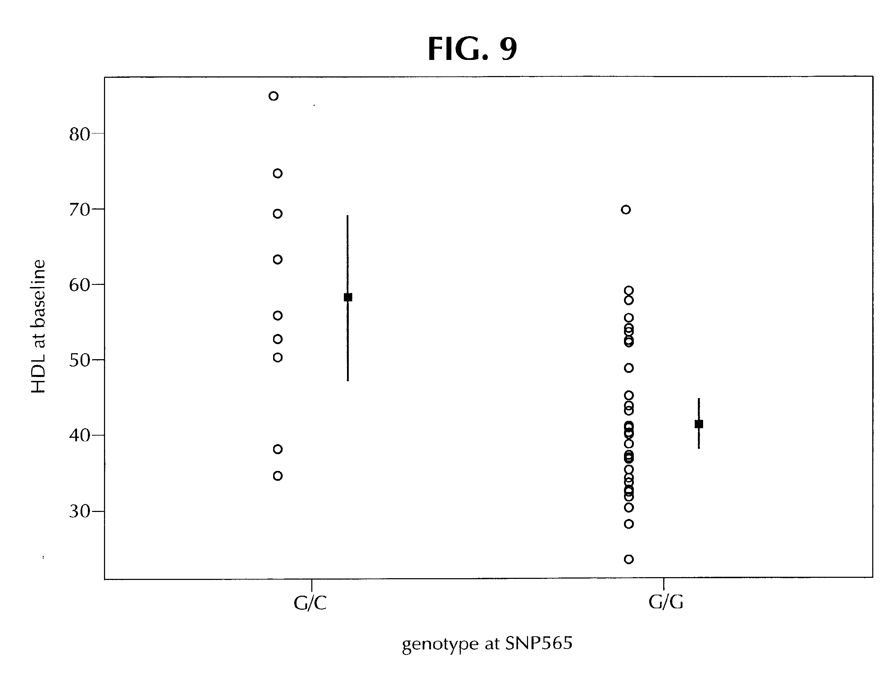

As shown in FIGURE 9, the presence of an allele containing a G to C mutation at the SNP 565 polymorphic site (and the corresponding insertion 307 in intron 12) of the CETP gene correlated with increased HDL levels (G/C > G/G).

As shown in FIGURE 10, the presence of an allele containing mutations at each of the Taq 1, Msp 1, Rsa 1 and SNP 565 (and the corresponding insertion 307 in intron 12) polymorphic sites (the 1121 haplotype) of the CETP gene correlated with decreased CETP concentrations in the subjects (1 > 0). 0 represents individuals that are homozygous for the mutant allele and 1 represents individuals that are heterozygous for the mutant allele.

As shown in FIGURE 11, the presence of an allele containing mutations at each of the Taq 1, Msp 1, Rsa 1 and SNP 565 (and the corresponding insertion 307 in intron 12) polymorphic sites (the 2212 haplotype) of the CETP gene correlated with increased HDL levels (1 > 0). 0 represents individuals that are homozygous for the wild-type allele and 1 represents individuals that are heterozygous for the wild-type allele.

### EQUIVALENTS

As will be apparent to those skilled in the art to which the invention pertains, the present invention may be embodied in forms other than those specifically disclosed above without departing from the spirit or essential characteristics of the invention. The particular embodiments of the invention described above, are, therefore, to be considered as illustrative and not restrictive. The scope of the invention is as set forth in the appendant claims rather than being limited to the examples contained in the foregoing description.

## Claims

1. A method for determining a whether a subject has a modified susceptibility to cardiovascular disease comprising: detecting in a nucleic acid sample from said subject at least one CETP allele selected from the group consisting of intron 1 (707); intron 8 (3707); intron 8 (3946); promoter (VNTR); insertion (307); intron 15 (493), wherein said CETP allele is associated with a modified level of CETP activity.

2. The method as defined in claim **1** wherein said cardiovascular disease is associated with low HDL.

3. The method as defined in claim **1** wherein said one or more alleles are selected from the group consisting of: intron **1** (707) allele 2; intron 8 (3707) allele 2; intron 8 (3946) allele 2; promoter (VNTR) allele 2; insertion (307) allele 2; intron 15 (493) allele 2, wherein detection of said allele indicates that the subject has a decreased predisposition to cardiovascular disease.

4. The method as defined in claim **1,** wherein said detecting utilizes a technique selected from the group consisting of: a) allele specific oligonucleotide hybridization; b) size analysis; c) sequencing; d) hybridization; e) 5' nuclease digestion; f) single-stranded conformation polymorphism; g) allele specific hybridization; h) primer specific extension; j) oligonucleotide ligation assay; and k) RFLP analysis.

5. The method as defined in claim **1,** further comprising amplifying the nucleic acid sample.

6. An isolated nucleic acid comprising at least 11 consecutive nucleotides of SEQ. ID. No: 6, 8, 10, 12 or 14 or a complement thereof.

7. An isolated nucleic acid comprising at least one GAAA repeat, or complement thereof, wherein said nucleic acid is amplified from the CETP promoter region corresponding to -2144 to -1974 nucleotides from the transcriptional start site.

8. A kit, comprising: a means for detecting one or more alleles at a CETP locus selected from the group consisting of: intron 1 (707); intron 8 (3707); intron 8 (3946); promoter (VNTR); insertion (307); and intron 15 (493), and a first primer oligonucleotide that hybridizes 5' or 3' to one of said CETP loci.

9. The kit as defined in claim **8**, further comprising a second primer oligonucleotide that hybridizes 5' or 3' to one of said CETP loci.

10. The kit as defined in claim **9**, wherein said first primer and said second primer hybridize to the same CETP loci and wherein said first primer and said second primer hybridize to opposite sides of a region in the range of between about 50 and about 1000 base pairs.

11. Use of at least one cardiovascular disorder therapeutic, wherein said at least one cardiovascular disorder therapeutic is selected by (i) detecting at least one CETP allele in a nucleic acid sample from a patient, (ii) diagnosing a cardiovascular disorder and (iii) selecting at least one cardiovascular disorder therapeutic, in the manufacture of a medicament for the treatment of said cardiovascular disorder.

12. Use according to claim **11** wherein said CETP allele is from a locus selected from the group consisting of: intron 1 (707); intron 8 (3707); intron 8 (3946); promoter (VNTR); insertion (307); and intron 15 (493).

13. A method for identifying a cardiovascular disorder therapeutic comprising: contacting a subject carrying at least one CETP allele selected from the group consisting of: intron 1 (707); intron 8 (3707); intron 8 (3946); promoter (VNTR); insertion (307); and intron 15 (493) with a test substance, and determining the effect of said test substance on CETP activity.

14. The method as defined in claim **13** wherein said subject is a transgenic animal or a cell.

15. A method for identifying a subject suffering from a cardiovascular disorder that would be responsive to treatment with at least one cardiovascular disorder therapeutic, comprising: detecting in a nucleic acid sample from said subject at least one CETP allele selected from the group consisting of intron 1 (707); intron 8 (3707); intron 8 (3946); promoter (VNTR); insertion (307); intron 15 (493), wherein said CETP allele is associated with a modified level of CETP acitivity.
